# EUROPEAN PATENT SPECIFICATION

(11) **EP 1 551 449 B1**
(45) Date of publication and mention of the grant of the patent: **06.10.2010**
(21) Application number: 03764547.0
(22) Date of filing: 14.07.2003
(51) Int. Cl.: A61K 39/00, A61K 39/385, G01N 33/53, C07K 17/00

(54) **REAGENTS AND METHODS FOR ENGAGING UNIQUE CLONOTYPIC LYMPHOCYTE RECEPTORS**
REAGENZIEN UND VERFAHREN ZUM EINGRIFF IN EINZIGARTIGE KLONOTYPE LYMPHOZYTEN-REZEPTOREN
REACTIFS ET PROCEDES PERMETTANT D'IMPLIQUER DES RECEPTEURS CLONOTYPIQUES DE LYMPHOCYTES UNIQUES

(30) Priority: 12.07.2002 US 395781 P
(43) Date of publication of application: 13.07.2005
(73) Proprietor: The Johns Hopkins University, Baltimore, MD 21201 (US)
(72) Inventor: SCHNECK, Jonathan, Silver Spring, MD 20902 (US); OELKE, Mathias, Baltimore, MD 21236 (US)
(74) Representative: Tombling, Adrian George
(86) International application number: PCT/US2003/021790
(87) International publication number: WO 2004/006951

(56) References cited:
- WO-A-02/065992
- WO-A-03/057171
- WO-A1-00/40968
- WO-A1-01/80833
- US-A- 3 140 113
- US-A- 6 015 884
- US-A- 6 015 884
- US-A- 6 140 113
- US-B1- 6 268 411
- US-B1- 6 269 411
- US-B1- 6 458 354
- MAUS M V ET AL: "Ex vivo expansion of polyclonal and antigen-specific cytotoxic T lymphocytes by artificial APCs expressing ligands for the T-cell receptor, CD28 and 4-1 BB" NATURE BIOTECHNOLOGY, NATURE PUBLISHING, US, vol. 20, no. 2, February 2002 (2002-02), pages 143-148, XP002225278 ISSN: 1087-0156
- LATOUCHE J-B ET AL: "Induction of human cytotoxic T lymphocytes by artificial antigen-presenting cells" NATURE BIOTECHNOLOGY, NATURE PUBLISHING, US, vol. 18, no. 4, April 2000 (2000-04), pages 405-409, XP002191046 ISSN: 1087-0156
- DEETHS MATTHEW J ET AL: "B7-1-dependent co-stimulation results in qualitatively and quantitatively different responses by CD4+ and CD8+ T cells" EUROPEAN JOURNAL OF IMMUNOLOGY, vol. 27, no. 3, 1997, pages 598-608, XP009056441 ISSN: 0014-2980
- OELKE M ET AL: "Generation and purification of CD8+ melan-A-specific cytotoxic T lymphocytes for adoptive transfer in tumor immunotherapy." CLINICAL CANCER RESEARCH : AN OFFICIAL JOURNAL OF THE AMERICAN ASSOCIATION FOR CANCER RESEARCH. MAY 2000, vol. 6, no. 5, May 2000 (2000-05), pages 1997-2005, XP002352660 ISSN: 1078-0432
- VON BERGWELT-BAILDON MICHAEL S ET AL: "Human primary and memory cytotoxic T lymphocyte responses are efficiently induced by means of CD40-activated B cells as antigen-presenting cells: Potential for clinical application" BLOOD, vol. 99, no. 9, 1 May 2002 (2002-05-01), pages 3319-3325, XP002352661 ISSN: 0006-4971
- OERTLI D ET AL: "ARTIFICIAL ANTIGEN-PRESENTING CELLS ENGINEERED BY RECOMBINANT VACCINIA VIRUSES EXPRESSING ANTIGEN, MHC CLASS II, AND COSTIMULATORY MOLECULES ELICIT PROLIFERATION OF CD4+ LYMPHOCYTES INVITRO" CLINICAL AND EXPERIMENTAL IMMUNOLOGY, OXFORD, GB, vol. 110, no. 1, October 1997 (1997-10), pages 144-149, XP000882604 ISSN: 0009-9104
- NAKAMURA MASAKI ET AL: "Dendritic cells genetically engineered to simultaneously express endogenous tumor antigen and granulocyte macrophage colony-stimulating factor elicit potent therapeutic antitumor immunity." CLINICAL CANCER RESEARCH : AN OFFICIAL JOURNAL OF THE AMERICAN ASSOCIATION FOR CANCER RESEARCH. AUG 2002, vol. 8, no. 8, August 2002 (2002-08), pages 2742-2749, XP002352662 ISSN: 1078-0432
- OELKE MATHIAS ET AL: "Ex vivo induction and expansion of antigen-specific cytotoxic T cells by HLA-Ig-coated artificial antigen-presenting cells." NATURE MEDICINE, vol. 9, no. 5, May 2003 (2003-05), pages 619-624, XP002352663 ISSN: 1078-8956
- PARDIGON N. ET AL: 'Delayed and separate costimulation in vitro supports the evidence of a transient " excited " state of CD8+ T cells during activation' THE JOURNAL OF IMMUNOLOGY vol. 164, 2000, pages 4493 - 4499
- BRUNMARK A.; O'ROURKE A.M.: 'Augmentation of mature CD4+ T cell responses to isolated antigenic Class II proteins by Fibronectin and Intercellular Adhesion Molecule-1' THE JOURNAL OF IMMUNOLOGY vol. 159, 1997, pages 1676 - 1685
- GAY D. ET AL: 'The Major Histocompatibility Complex-restricted antigen receptor on T cells' THE JOURNAL OF IMMUNOLOGY vol. 136, no. 6, 1986, pages 2026 - 2032
- MOTTA I. ET AL: 'In vitro induction of naive cytotoxic T lymphocytes with complexes of peptide and recombinant MHC class I molecules coated onto beads: role of TCR/ligand density' EUROPEAN JOURNAL OF IMMUNOLOGY vol. 28, 1998, pages 3685 - 3695
- MARTI W.R. ET AL: 'Induction of antigen-presenting capacity in tumor cells upon infection with non-replicating recombinant vaccinia virus encoding MHC class II and costimulatory molecules' JOURNAL OF IMMUNOLOGICAL METHODS vol. 200, 1997, pages 191 - 198
- SPRENT J. ET AL: 'Constructing artificial antigen-presenting cells from Drosophila cells', 1997, DENDRITIC CELLS IN FUNDAMENTAL AND CLINICAL IMMUNOLOGY;RICCIARDI-CASTAGNOLI ED.; PLENUM PRESS PUB., NEW YORK

### SEQUENCE LISTING

<110> The Johns Hopkins University
<120> Reagents and Methods for Engaging Unique Clonotypic Lymphocyte Receptors
<130> 001107.00341
<150> US 60/395,781
   <151> 2002-07-12
<160> 4
   <170> FastSEQ for Windows Version 4.0
<210> 1
   <211> 18
   <212> PRT
   <213> Artificial Sequence
<220>
   <223> peptide linker
<400> 1
   Gly Leu Tyr Gly Leu Tyr Gly Leu Tyr Thr His Arg Ser Glu Arg Gly
    1               5                  10                  15
   Leu Tyr
<210> 2
   <211> 17
   <212> PRT
   <213> Artificial Sequence
<220>
   <223> peptide linker
<400> 2
   Gly Leu Tyr Ser Glu Arg Leu Glu Gly Leu Tyr Gly Leu Tyr Ser Glu
    1               5                  10                  15
   Arg
<210> 3
   <211> 10
   <212> PRT
   <213> Artificial Sequence
<220>
   <223> peptide linker
<400> 3
   Glu Leu Ala Gly Ile Gly Ile Leu Thr Val
   1               5                   10
<210> 4
   <211> 9
   <212> PRT
   <213> Artificial Sequence
<220>
   <223> peptide linker
<400> 4
   Asn Leu Val Pro Met Val Ala Thr Val
   1               5

## Description

### FIELD OF THE INVENTION

The invention relates to reagents and methods for engaging unique clonotypic lymphocyte receptors.

### BACKGROUND OF THE INVENTION

Development of immunotherapy, both adoptive and active, has been impeded by the lack of a reproducible, economically viable method to generate therapeutic numbers of specific T or B lymphocytes. For example, the current standard approach to generating antigen-specific cytotoxic T lymphocytes (CTL) for adoptive immunotherapy entails generating monocyte-derived dendritic cells (DC) for expansion of CTL. This step is both time consuming and expensive. Use of DC for CTL expansion to clinically relevant amounts of CTL requires multiple leukaphereses to obtain enough autologous DC. Variability seen with both the quantity and quality of DC obtained, which presumably relates to underlying disease and patient pretreatment, also significantly impacts on the viability of DC-based *ex vivo* therapeutics. For these reasons, use of DC has been a limiting step in *ex vivo* expansion of T cells.

Other approaches for expansion of antigen-specific CTL from enriched populations have used nonspecific anti-CD3 based techniques. Levine et al., J Hematother. 7, 437-48, 1998. However two problems arise. First, anti-CD3/anti-CD28 beads support long-term growth of CD4 T cells, but do not sustain long term growth of CD8 T cells. Deeths & Mescher, Eur. J. Irnmunol. 27, 598-608, 1997. In addition, approaches using anti-CD3 based stimulation are associated with a decrease in antigenic specificity even when starting with highly enriched antigen-specific CTL populations. Maus et al., Nature Biotechnol. 20, 143-48, 2002. These problems substantially limit the delivery of therapeutically relevant lymphocytes.

Pardigon N. et al., (J. Immunol. 164, 4493-4499, 2000) discloses a rigid solid support (flat bottom 96-well plastic plates) coated with recombinant single-chain MHC class I molecules, loaded with the HA peptide of the influenza virus, or with "altered peptide 6G", and dimerized with a K^{d}-specific antibody. However, this system does not allow the long term expansion of antigen-specific CD8⁺ T cells.

There is, therefore, a need in the art for effective means of generating therapeutically useful populations of antigen-specific T cells, as well as specific antibody-producing B cells.

### BRIEF SUMMARY OF THE INVENTION

The invention provides at least the following embodiments. One embodiment of the invention is a solid support comprising (A) an antibody that specifically binds to CD28; and (B) an MHC class I immunoglobulin complex comprising:
(1) two fusion proteins, wherein each fusion protein comprises an MHC class I α chain comprising a peptide binding groove, and an immunoglobulin heavy chain comprising a variable region; wherein the immunoglobulin heavy chain is C-terminal to the MHC chain; and wherein the immunoglobulin heavy chains of the two fusion proteins associate to form the MHC class I immunoglobulin complex, wherein the MHC class I immunoglobulin complex comprises a first MHC class I peptide binding cleft and a second MHC class I binding cleft;
(2) two MHC class I β₂ microglobulin polypeptides; and
(3) two immunoglobulin light chains.

A further embodiment of the invention provides a preparation comprising a plurality of particles. Particles of the plurality comprise (A) at least one B cell affecting molecule and (B) at least one molecular complex that engages B cell surface immunoglobulins or MHC-antigen complexes on a B cell surface.

Still another embodiment of the invention provides a method of inducing the formation of antigen-specific T cells. An isolated preparation comprising a plurality of precursor T cells is contacted with at least one first solid support. The solid support comprises at least one T cell affecting molecule and at least one antigen presenting complex that comprises at least one antigen binding cleft. An antigen is bound to the antigenic binding cleft. Members of the plurality of precursor T cells are thereby induced to form a first cell population comprising antigen-specific T cells that recognize the antigen. The number or percentage of antigen-specific T cells in the first population is greater than the number or percentage of antigen-specific T cells that are formed if precursor T cells are incubated with a solid support that comprises an antibody that specifically binds to CD3 but does not comprise an antigen presenting complex. The antigen-specific T cells can be administered to a patient.

Yet another embodiment of the invention provides an *in vitro* method of increasing the number or percentage of antigen-specific T cells in a population of cells. A first cell population comprising antigen-specific T cells is incubated with at least one first solid support. The solid support comprises at least one T cell affecting molecule and at least one antigen presenting complex that comprises at least one antigen binding cleft. An antigen is bound to the antigenic binding cleft. The step of incubating is carried out for a period of time sufficient to form a second cell population comprising an increased number or

The invention thus provides a variety of reagents and methods for engaging unique clonotypic lymphocyte receptors. The invention also provides reagents and methods for obtaining antigen-specific T cells, which can be used for therapeutic purposes.

### BRIEF DESCRIPTION OF THE FIGURES

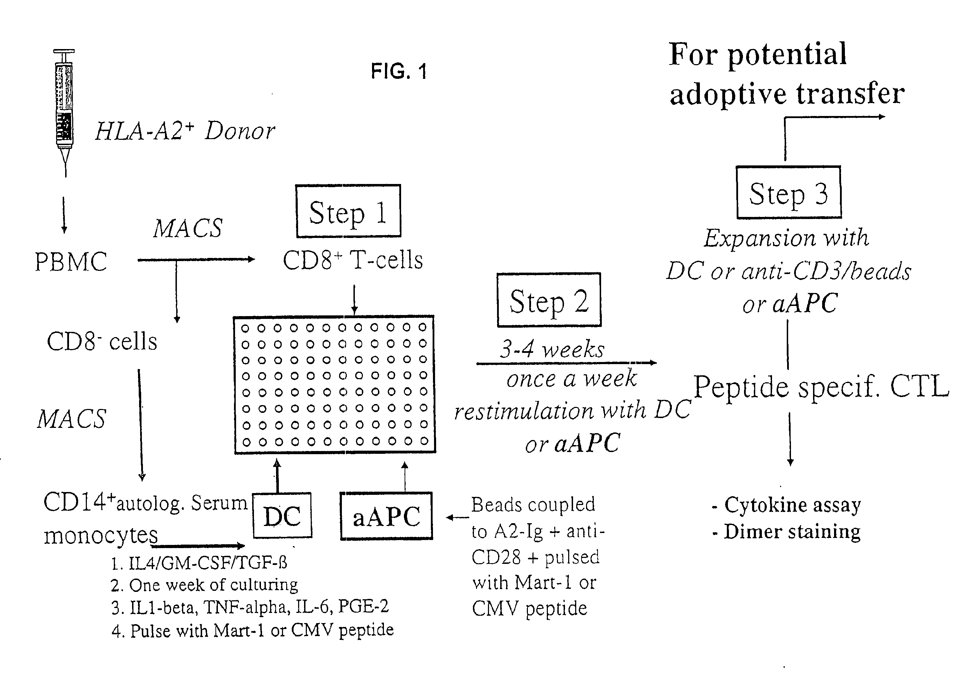

FIG. 1. Schematic of induction and expansion of peptide-specific CTL by either autologous DC or aAPC.

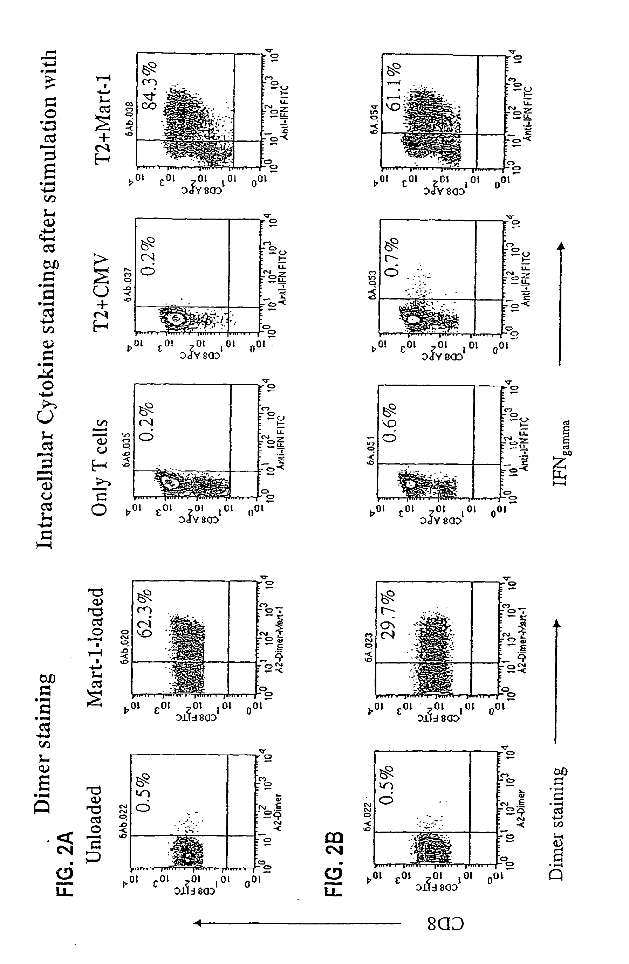

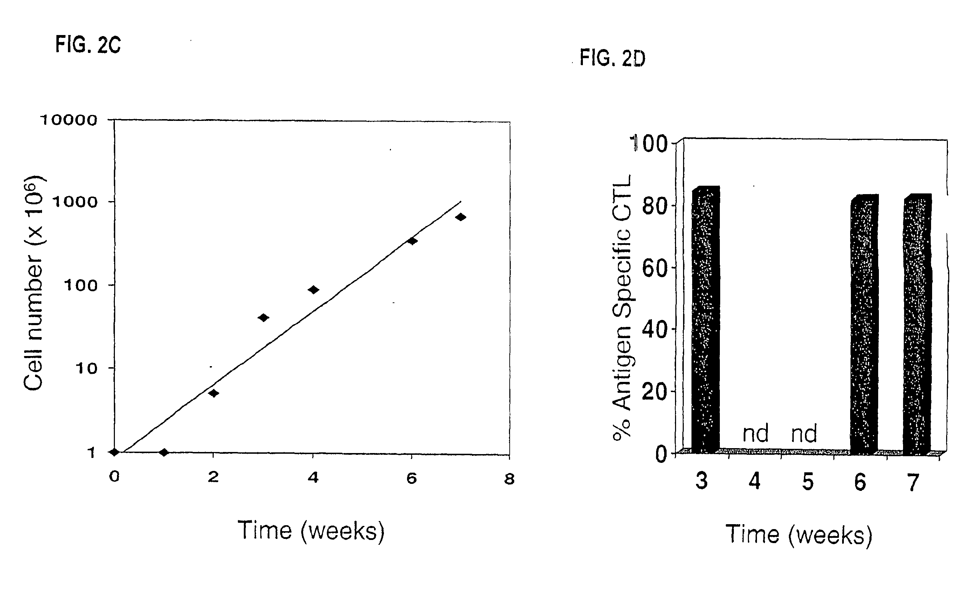

FIG. 2. Induction and growth potential of Mart-1-specific CD8⁺ T cells stimulated with aAPC. FIG. 2A, results of stimulation with aAPC. FIG. 2B, results of stimulation with DC.

FIG. 2C, graph showing expansion of T cells. FIG. 2D, graph showing percentage of antigen-specific CTL in expanded T cell population.

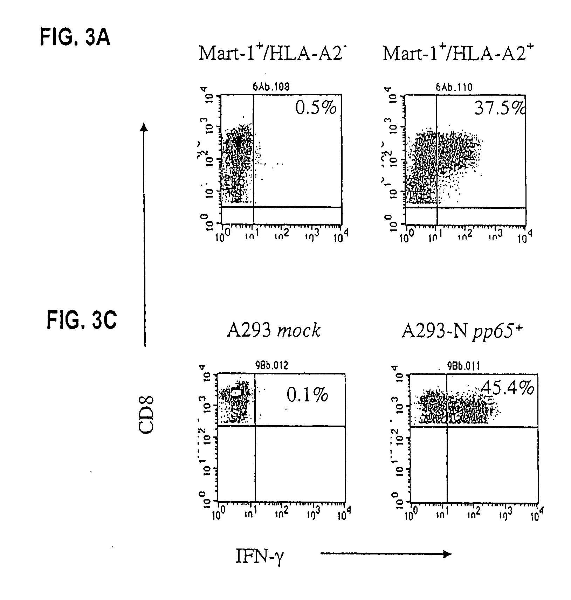

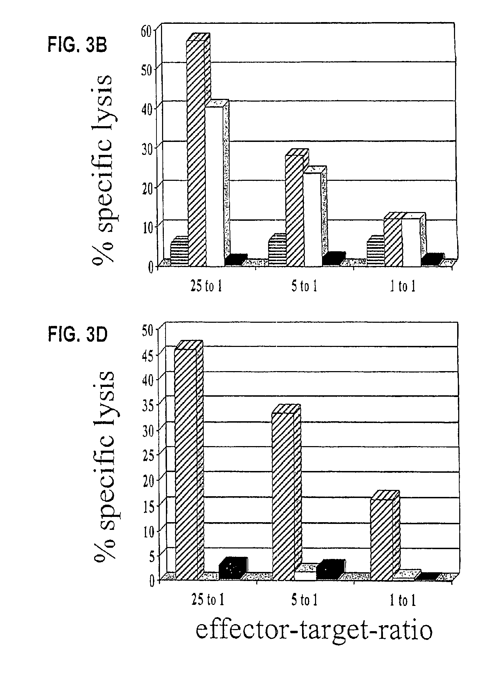

FIG. 3. aAPC-induced antigen-specific CTL recognize endogenous melanoma or pp65 antigen on target cells. FIG. 3A, Percentage of peptide-specific, CD8⁺ T cells is shown for Mart-1 specific T cells stimulated with a Mart-1⁺/HLA-A2⁻Melanoma cell line (left) or with a Mart-1⁺/HLA-A2⁺ Melanoma cell line (right). FIG. 3B, Percent specific lysis by a Mart-1 specific CTL line is shown for the following targets: T2 cells pulsed with either non specific CMV peptide ( 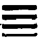 ), or specific Mart-1 peptide ( 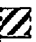 ), or with either an allogeneic HLA-A2⁺ melanoma cell line ( □ ), or an allogeneic HLA-A2⁻ melanoma cell line (■ ). Values represent triplicates at effector-target-ratios of 25:1, 5:1 and 1:1. FIG. 3C, Percentage of peptide-specific, CD8⁺ T cells is shown for CMV specific T cells stimulated with either a pp65⁻ control transfected HLA-A2⁺ A293 cells (left) or with a pp65⁺ transfected HLA-A2⁺ A293 cells (right). FIG. 3D, ⁵¹Cr-release assay results for CMV specific CTL cytotoxic activity against target cells expressing endogenous antigen. Percent specific lysis by a CMV specific CTL line is shown for the following targets: pp65 transfected A293 cells ( 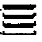 ), nontransfected HLA-A2⁺ A293 cells (□ ) and with IE (intermediate early protein from CMV) control transfected A293 cells ( ■ ). The antigen specific CD8⁺ T cells for all assays were obtained after 3-7 weeks *in vitro* culture with peptide loaded aAPC.

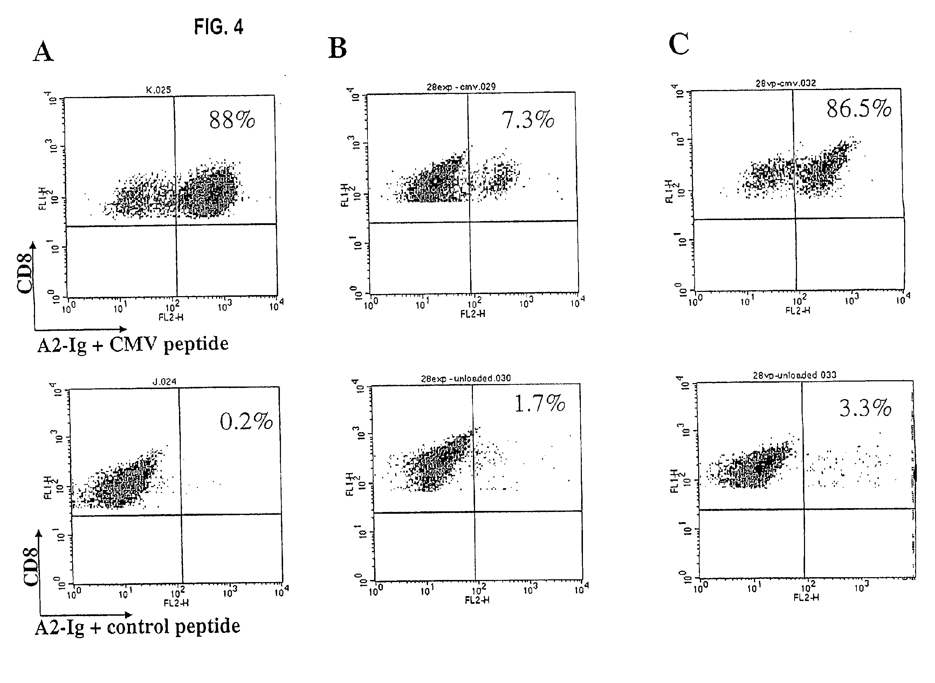

FIG. 4. Frequency of antigen-specific CTL after expansion with anti-CD3 beads or aAPC. T cells were isolated and purified as described in Example 1. FIG. 4A, T cells stimulated with autologous, monocyte-derived DC-pulsed with CMV peptide to induce antigen-specific T cell expansion. FIG. 4B, after three weeks of induction, T cell populations were expanded on anti-CD3/anti-CD28 beads. FIG. 4C, after three weeks of induction on DC, T cell populations were expanded on peptide-loaded HLA-Ig based aAPC. In both cases, approximately 7-fold expansion was seen after 10 days of culture. Cells were stained with FITC-conjugated anti-CD8 mAb and CMV-peptide-pulsed A2-Ig loaded with pp65 (top panels) or with A2-Ig loaded with a control peptide, Mart-1, as described in Example 1. The percent of peptide-specific CD8⁺ CTL is shown in the upper right corner.

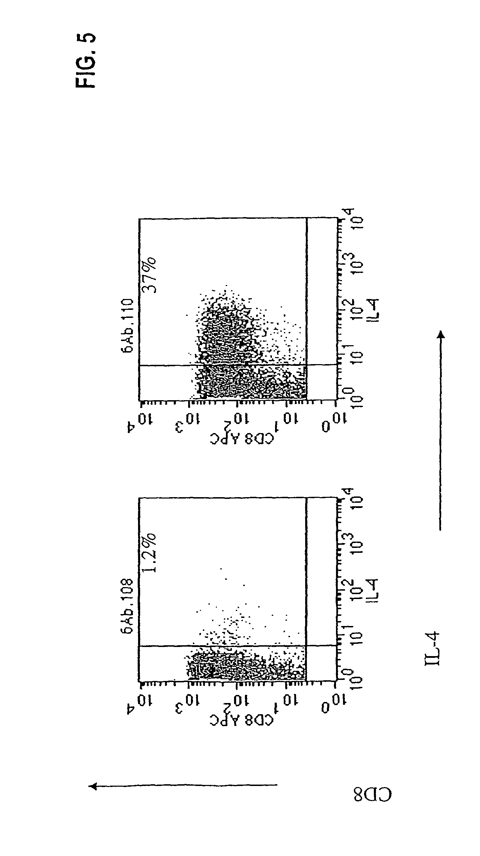

FIG. 5. aAPC-induced Mart-1 CTL recognize endogenous antigen on melanoma target cells. Mart-1 specific CD8⁺ cells were obtained after *in vitro* culture with Mart-1 loaded aAPC. Mart-1-specific T cells were stimulated with either a Mart-1⁺/HLA-A2⁻ melanoma cell line (1^{st} column) or with a Mart-1⁺/HLA-A2⁺ Melanoma cell line (2^{nd} column). For the ICS staining the cells were incubated with melanoma cells in regular medium without cytokines. To elevate the baseline, a low dose of PMA and Ionomycin was added to the medium. After one hours, Monensin (Golgi-stop) was added to the culture. After six hours, the T cells were harvested and analyzed by intracellular cytokine staining. The percentage of peptide-specific, IL-4⁺/CD8⁺ T cells is shown.

### DETAILED DESCRIPTION OF THE INVENTION

The invention provides a wide variety of tools and methods for engaging (*i*.*e*., binding and triggering a physiological response) unique clonotypic lymphocyte receptors. Unique clonotypic receptors include, for example, T cell receptors that recognize a specific antigen. Some embodiments of the invention ("antigen presenting platforms and methods") can be used to induce formation and/or expansion of antigen-specific T cells for therapeutic or diagnostic purposes. Antigen-specific T cells include cytotoxic T lymphocytes, helper T cells (*e*.*g*., Th1, Th2), and regulatory T cells. Still other embodiments of the invention ("antibody inducing platforms and methods") can be used to induce the formation and/or expansion of B lymphocytes that produce antibodies directed against particular antigens.

### Antigen presenting platforms and methods

Antigen presenting platforms of the invention (also referred to herein as "artificial antigen presenting cells" or "aAPCs"), as described in more detail below, can be based on eukaryotic cells or artificial solid supports. Antigen presenting platforms of the invention comprise at least one T cell affecting molecule (*e.g*., a T cell costimulatory molecule, a T cell growth factor, an adhesion molecule, a regulatory T cell inducer molecule, or an apoptosis-inducing molecule) and at least one antigen presenting complex.

### Antibody inducing platforms

Antibody inducing platforms of the invention, as described in more detail below, also can be based on eukaryotic cells or artificial solid supports. Antibody inducing platforms of the invention comprise at least one B cell affecting molecule (*e.g*., CD40 ligand, a cytokine, or a cytokine molecular complex, described below) and at least one molecular complex that engages B cell surface immunoglobulins or engages MHC-antigen complexes on the surface of a B cell.

Use of antigen presenting and antibody inducing platforms of the invention for *ex vivo* expansion of antigen-specific T cells and antibody-specific B cells, respectively, has a number of important advantages over currently used methods. Both types of platforms can be preformed, have reproducible antigen presenting or antibody inducing activity, and can be used for a large patient population. The use of antigen presenting platforms dramatically simplifies and shortens the *ex vivo* expansion process of antigen-specific T cells compared to current methods using dendritic cells. In addition, the antigen-specific T cell population expanded with these platforms will contain up to 80% antigen-specific T cells compared to 5-20% obtained with current methods (*e.g*., stimulation with anti-CD3 antibody alone). The platforms can induce expansion of precursor T or B cells to numbers suitable for therapeutic use. The platforms can combine precursor T or B cell isolation with antigen-specific stimulation in one step. Embodiments of the platforms based on artificial particles are superior to currently available means of inducing specific T or B cells populations in that they can be of high-density and can settle by gravity, they can have magnetic properties if separation by magnet is desired, they have ideal surface chemistry for coating and protein conjugation, and different particle sizes and geometry are available to provide for increased surface area and increased contact with target cells.

Components of platforms of the invention are described in detail below.

### Solid supports

Solid supports for platforms of the invention can be any solid, artificial surface (*i.e*., non-cell) to which protein molecules can be attached. Suitable solid supports include rigid supports (*e.g*., flasks, tubes, culture dishes, multi-well plates, slides, particles) as well as flexible supports (*e.g*., infusion bags).

### Flexible supports

Flexible supports include infusion bags. The bags can be produced for single-use or can be reusable. Preferably bags are made of a material suitable for sterilization. Such materials are well-known and widely used in the art.

### Rigid supports

Examples of rigid supports include tubes; tissue culture vessels, such as flasks (*e.g.,* 10, 25, 75, 150, 285, 300, or 420 cm²), petri dishes (*e.g*., 9.2, 22.1, 60, 147.8 cm²), multi-well plates (*e.g.,* 6-, 12-, 24-, 48-, or 96-, or 384-well plates); slides; and particles. Rigid supports can be made, for example, out of metals such as iron, nickel, aluminum, copper, zinc, cadmium, titanium, zirconium, tin, lead, chromium, manganese and cobalt; metal oxides and hydrated oxides such as aluminum oxide, chromium oxide, iron oxide, zinc oxide, and cobalt oxide; metal silicates such as of magnesium, aluminum, zinc, lead, chromium, copper, iron, cobalt, and nickel; alloys such as bronze, brass, stainless steel, and so forth. Rigid supports can also be made of non-metal or organic materials such as cellulose, ceramics, glass, nylon, polystyrene, rubber, plastic, or latex. Alternatively, rigid supports can be a combination of a metal and a non-metal or organic compound, for example, methacrylate- or styrene-coated metals and silicate coated metals. The base material can be doped with an agent to alter its physical or chemical properties. For example, rare earth oxides can be included in aluminosilicate glasses to create a paramagnetic glass materials with high density (see White & Day, Key Engineering Materials Vol. 94-95, 181-208, 1994).

### Particles

In one set of embodiments, platforms of the invention are based on artificial particles. Artificial particles can be made of any of the numerous materials described above. If desired, particles can be made entirely of biodegradable organic materials, such as cellulose, dextran, and the like. Suitable commercially available particles include, for example, nickel particles (Type 123, VM 63, 18/209A, 10/585A, 347355 and HDNP sold by Novamet Specialty Products, Inc., Wyckoff, N.J.; 08841R sold by Spex, Inc.; 01509BW sold by Aldrich), stainless steel particles (P316L sold by Ametek), zinc dust (Aldrich), palladium particles (D13A17, John Matthey Elec.), M-450 Epoxy Beads (Dynal), and TiO₂, SiO₂, or MnO₂ particles (Aldrich).

The density of particles can be selected such that the particles will differentially settle through a sample suspension more rapidly than cells. Thus, particles preferably are composed of a high-density material to facilitate cell separation and manipulation of the particles. Use of such particles permits the particles to settle under gravity to facilitate their separation from antigen-specific T cells, T cell precursors, B cell precursors, B cells, or other cells.

A further advantage of using particles of high density is that large quantities of non-target cells can be purged without losing target cells, which is useful for therapeutic applications. Multiple cell separation cycles can be performed as described by Kenyon et al. ("High Density Particles: A Novel, Highly Efficient Cell Separation Technology," in CELL SEPARATION METHODS AND APPLICATIONS, Recktenwald & Radbruch, eds., Marcel Dekker, Inc., 2000, pp. 103-32), such that only 2-3% nonspecific cell loss occurs per depletion cycle. Using a multiple cycle approach, non-target cells can be purged from a blood product without significant loss of target cells (*e*.*g*., T or B cell precursors). Recovery of target cells can be greater than 90%. For example, particles of high density can reduce normal B cells in mobilized apheresis products by an average of 4.7 logs but retain greater than 90% of the CD34+ cells in a system that used three depletion cycles. Houde et al., Blood 96, 187a, 2000.

In one embodiment, particles are nickel particles (*e.g*., Type 123 nickel particles from Novamet, which range in size from 3 to 7 µm) that have a density of approximately 9 gm/km³ and are magnetic. Unlike other commercially available particles for which a magnet must be used to capture particle-target cell complexes, high density nickel particles settle by gravity. After settling, a magnet can be used to separate unwanted particles from cells in a suspension. Nickel particles also have chemical properties that permit the attachment of a variety polymers and inorganic molecules with functional moieties that are useful for ligand coupling chemistry.

The configuration of particles can vary from being irregular in shape to being spherical and/or from having an uneven or irregular surface to having a smooth surface. Preferred characteristics of particles can be selected depending on the particular conditions under which the antigen presenting platforms will be prepared and/or used. For example, spherical particles have less surface area relative to particles of irregular size. If spherical particles are used, less reagent is necessary due to the reduced surface area. On the other hand, an irregularly shaped particle has a significantly greater surface area than a spherical particle, which provides an advantage for conjugated protein content per surface area and surface area contact for cells.

The size of particles also can vary. The particle size (nominal diameter) is not critical to the invention but will typically range from 0.05-50 µm, more typically 3-35 µm, and is preferably about 5 µm. The particles can be uniform in size or can vary in size, with the average particle size preferably being in the range of 0.05-50 µm. Other particles can be finely divided powders or ultrafine particles. Particles of nickel powder with a nominal diameter of about 5 microns have excellent protein adsorption properties. In one embodiment, the particles have a surface area of at least 0.4 m²/g, preferably from about 0.4 m²/g to about 0.5 m²g. Particle size distribution can be conveniently determined, for example, using a Microtrak instrument based on dynamic light scattering.

### Coating of solid supports

A solid support can be coated before proteins are bound to its surface. Once a coating chemistry has been chosen, the surface of a solid support can be activated to allow the specific attachment of particular protein molecules. Thus, coatings can be selected with a view to optimal reactivity and biocompatibility with various T or B cell populations or T or B precursor cell populations. Preferably, whatever coating chemistry is used provides a suitable matrix for further activation chemistry. Numerous such coatings are well known in the art. For example, solid supports can be coated with human serum albumin, tris (3-mercaptopropyl)-N-glycylamino) methane (U.S. Patent 6,074,884), gelatin-aminodextrans (U.S. Patent 5,466,609), or amino acid homopolymers or random copolymers. In one embodiment, a random amino acid copolymer comprising poly(glutamate, lysine, tyrosine) [6:3:1] is used; this copolymer is available from Sigma Chemical Co. as Product No. P8854. It is a linear random polymer of the amino acids glutamic acid, lysine, and tyrosine in a ratio of 6 parts glutamic acid, 3 parts lysine, and 1 part tyrosine. In another embodiment, an amino acid copolymer is used that includes lysine and tyrosine in a ratio of 4 parts lysine to 1 part tyrosine. In yet another embodiment, an amino acid copolymer is used that includes lysine and alanine in a ratio of 1 part lysine to 1 part alanine.

In another embodiment, a solid support is coated with a synthetic polymer, then the synthetic polymer is activated before it is linked to a protein molecule including, but not limited to, a T or B cell affecting molecule, an antigen presenting complex, or a molecular complex that engages B cell surface immunoglobulins or MHC-antigen complexes on a B cell surface.

### Coating with Silica (SiO₂)

In another embodiment, particularly well suited for nickel surfaces (especially particles), a solid support is coated with silica. A silica surface has several advantages over the more commonly used organic polymer surfaces. It is highly uniform, chemically defined, and chemically and thermally stable, with silanol residues covering the entire surface and available for stable covalent coupling with amino- or epoxy- derivatives of triethoxysilanes for attaching proteins and other biomolecules. Silane derivatives can cover the entire surface, forming a monolayer of a two-dimensional polymer that permits a high degree of control over specific and non-specific interactions on the surface.

Methods for coating various solid supports with silica are disclosed in U.S. Patent 2,885,399; see also Birkmeyer et al., Clin Chem. 1987 Sep;33(9):1543-7. For example, a solid support can be incubated with a solution of sodium metasilicate, sodium aluminate, and boric acid to form polymerized silica that deposits on the surface. Another method of silica coating is to mix sodium silicate with the solid support and lower the pH with sulfuric acid at 95°C, followed by water washes. See U.S. Patent 2,885,366; Eagerton, KONA 16, 46-58, 1998. For example, nickel surfaces can be coated by first dispersing them in a 0.2 N NaSO₄ solution and heating the solution to 95°C. The pH is adjusted to 10 with NaOH. Sodium silicate in sulfuric acid is then added and mixed at 95°C for 0.5 hours. The support is washed several times with distilled water. The extent of coating can be examined by determining the resistance of the support to nitric acid digestion.

ESCA analysis for surface chemical composition, which is based on X-ray scattering, can be used to obtain the elemental composition of a support surface, providing information on the degree of surface coating and silanation with active residues.

### Coating with Aluminum Oxide

In another embodiment, a surface matrix on a solid support is provided by "passivating" a nickel surface with a non-toxic metal oxide coating, such as aluminum oxide. Other methods of coating include depositing metal oxides such as aluminum oxide to the surface of the solid support. Aluminum oxide is a useful matrix because it provides an inert surface with low nonspecific binding properties that can be functionalized for protein conjugation.

An aluminum oxide coating can be provided by a number of methods, such as the sol-gel process, in which a thin, continuous layer of amorphous aluminum oxide is formed by evaporation of an aluminum sol-gel onto the solid support, followed by baking in air to form the oxide. Ozer et al, SPIE 3 789, 77-83, 1999. In other embodiments, conventional physical vapor deposition techniques (Smidt, Inter Mat Rev 35, 21-27, 1990) or chemical vapor deposition (Koh et al., Thin Solid Films 304, 222-24, 1997) can be used. If a nickel solid support is used, the thickness of such coatings can be controlled to provide adequate stability while minimizing nickel leaching. The success of sealing the nickel can be tested by quantitative chemical assays of nickel ions. Solid supports can be incubated at various temperatures in various buffers and biological fluids, and the levels of nickel ions in these media can be measured.

### Surface Coating Efficiency

The completeness of a surface coating can be determined through surface leaching assays. For example, when the surface of a nickel solid support is completely coated by glass or other non-reactive metal, the solid support is resistant to nickel leaching under acidic conditions. For example, a known mass of coated nickel solid supports can be incubated in 10% nitric acid and observed for 24 hours. As nickel is dissolved the solution turns green. Untreated nickel turns the solution green immediately. Nickel solid supports that have a nickel oxide layer on their surface turn the solution green in about 20 minutes. Solid supports coated with a layer of silica as described above are resistant to nitric acid for greater than 8 hours, which indicates that a thick layer of silica deposited on the surface. Solid supports can also be tested in aqueous conditions by incubating the supports in cell culture medium similar to the culture conditions used for B or T cell activation (described below). The amount of nickel leached into the solution can be measured by atomic absorption spectrometry.

### Pretreatment before coating

If desired, solid supports can be pre-treated before being coated. Pre-treatment of a solid support, for example, can sterilize and depyrogenated the support, as well as create an oxide layer on the support's surface. This pretreatment is particularly beneficial when metallic solid supports are used. In one embodiment, pre-treatment involves heating a nickel solid support for about 2-6 hours, preferably for about 5 hours, at a temperature within the range of about 200-350°C, preferably about 250 °C.

### Attachment ofprotein molecules to solid supports

Molecules can be directly attached to solid supports by adsorption or by direct chemical bonding, including covalent bonding. *See, e*.*g.,* Hermanson, BIOCONJUGATE TECHNIQUES, Academic Press, New York, 1996. A molecule itself can be directly activated with a variety of chemical functionalities, including nucleophilic groups, leaving groups, or electrophilic groups. Activating functional groups include alkyl and acyl halides, amines, sulfhydryls, aldehydes, unsaturated bonds, hydrazides, isocyanates, isothiocyanates, ketones, and other groups known to activate for chemical bonding. Alternatively, a molecule can be bound to a solid support through the use of a small molecule-coupling reagent. Non-limiting examples of coupling reagents include carbodiimides, maleimides, N-hydroxysuccinimide esters, bischloroethylamines, bifunctional aldehydes such as glutaraldehyde, anyhydrides and the like. In other embodiments, a molecule can be coupled to a solid support through affinity binding such as a biotinstreptavidin linkage or coupling, as is well known in the art. For example, streptavidin can be bound to a solid support by covalent or non-covalent attachment, and a biotinylated molecule can be synthesized using methods that are well known in the art. See, for example, Hermanson, 1996.

If covalent binding to a solid support is contemplated, the support can be coated with a polymer that contains one or more chemical moieties or functional groups that are available for covalent attachment to a suitable reactant, typically through a linker. For example, amino acid polymers can have groups, such as the ε-amino group of lysine, available to couple a molecule covalently via appropriate linkers. The invention also contemplates placing a second coating on a solid support to provide for these functional groups.

### Activation chemistries

Activation chemistries can be used to allow the specific, stable attachment of molecules to the surface of solid supports. There are numerous methods that can be used to attach proteins to functional groups; see Hermanson, 1996. For example, the common cross-linker glutaraldehyde can be used to attach protein amine groups to an aminated solid support surface in a two-step process. The resultant linkage is hydrolytically stable. Other methods include use of cross-linkers containing n-hydro-succinimido (NHS) esters which react with amines on proteins, cross-linkers containing active halogens that react with amine-, sulfhydryl-, or histidine- containing proteins, cross-linkers containing epoxides that react with amines or sulfhydryl groups, conjugation between maleimide groups and sulfhydryl groups, and the formation of protein aldehyde groups by periodate oxidation of pendant sugar moieties followed by reductive amination.

In one embodiment, protein molecules are attached to a silica coating using 3-aminopropyltriethoxysilane (Weetall & Filbert, Methods Enzymol. 34, 59-72, 1974). This compound forms a stable covalent bond with a silica surface and at the same time renders the surface more hydrophobic. The silanation reaction can be conducted in an aqueous low pH medium, which is known to allow the formation of a monolayer with the amino groups available for conjugation. The attachment of proteins can be via the homobifunctional coupling agent glutaraldehyde or by a heterobifunctional agents such as SMCC. After protein attachment, residual surface-associated coupling agents can be activated by incubating with various proteins, hydrophilic polymers, and amino acids. Albumin and polyethylene glycols are particularly suitable because they block non-specific binding of proteins and cells to solid phases.

In another embodiment, aminosilanation is used to activate the surface of aluminum oxide-coated solid supports. See U.S. Patent 4,554,088 1985. Another method of activating the surface of the aluminum oxide coated solid supports is to adsorb a strongly adhering polymer, such as a glu-lys-tyr tripeptide. The tripeptide polymer can be activated through the lysine amines by reaction with a homobifunctional cross-linker, such as difluorodinitrobenzene, or by reaction with glutaraldehyde. Proteins can then be attached directly to the activated surface.

### Optimization of Functional Protein Conjugation

The attachment of specific proteins to a solid support surface can be accomplished by direct coupling of the protein or by using indirect methods. Certain proteins will lend themselves to direct attachment or conjugation while other proteins or antibodies retain better functional activity when coupled to a linker or spacer protein such as anti-mouse IgG or streptavidin. If desired, linkers or attachment proteins can be used.

### Optimization of Ratio of Functional Proteins Coupled to Solid Supports

The ratio of particular proteins on the same solid support can be varied to increase the effectiveness of the solid support in antigen or antibody presentation. For example, Optimum ratios of A2-Ig (described in Example 1, below) (Signal 1) to anti-CD28 (Signal 2) can be tested as follows. Solid supports are coupled with A2-Ig and anti-CD28 at a variety of ratios, such as 30:1, 10:1, 3:1, 1:1, 0.3:1; 0.1:1, and 0.03:1. The total amount of protein coupled to the supports is kept constant (for example, at 150 mg/ml of particles) or can be varied. Because effector functions such as cytokine release and growth may have differing requirements for Signal 1 versus Signal 2 than T cell activation and differentiation, these functions can be assayed separately.

### Analytical Assays

Solid supports can be characterized by several analytical assays to evaluate the additions and reactions taking place as supports are produced. These include assays for functional groups, such as amines and aldehydes, and assays for the binding of particular types of protein molecules. In addition, functional assays can be used to evaluate biological activity of the solid supports. The amount of protein bound to the surface of solid supports can be determined by any method known in the art. For example, bound protein can be measured indirectly by determining the amount of protein that is removed from the reaction solution using absorbance at 280 nm. In this embodiment, the protein content of the reaction solution before and after addition to the solid support is measured by absorbance at 280 nm and compared. The amount of protein contained in any wash solutions is also measured and added to the amount found in the post reaction solution. The difference is indicative of the amount bound to the surface of the solid support. This method can be used to rapidly screen for binding efficiency of different reaction conditions.

In another embodiment, the amount of protein bound to solid supports can be measured in a more direct assay by binding assays of labeled antigens and antibodies. For example, various concentration of antibody-conjugated solid supports can be incubated with a constant concentration of HRP-labeled antigen or goat-anti-mouse IgG. The supports are washed in buffer to remove unbound labeled protein. Measuring the support-associated HRP using OPD substrate gives the concentration of bound labeled protein. A Scatchard Plot analysis can provide the concentration and affinity of the immobilized proteins. HRP-labeled antibodies can be obtained commercially or antibodies can be labeled with HRP using the glutaraldehyde method of Avrameas & Ternync, Immunochemistry 8, 1175-79, 1971.

The methods described above measure both covalently bound and non-covalently bound protein. To distinguish between the two types of binding, solid supports can be washed with a strong chaotrope, such as 6 M guanidine hydrochloride or 8 M urea. Non-specific binding is disrupted by these conditions, and the amount of protein washed off the solid supports can be measured by absorbance at 280 nm. The difference between the total amount of protein bound and the amount washed off with the chaotrope represents the amount of protein that is tightly bound and is likely to be covalently attached.

### Cells

Both antigen presenting platforms and antibody inducing platforms of the invention can be based on cells. The cells preferably are eukaryotic cells, more preferably mammalian cells, even more preferably primate cells, most preferably human cells.

Many of the molecules on the surface of platforms of the invention have been cloned. Thus, cells can be transfected with constructs encoding such molecules. Methods of transfecting cells are well known in the art and include, but are not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, and calcium phosphate-mediated transfection.

Alternatively, proteins can be chemically bound to the cell surface. Any methods of coupling a protein to a cell surface can be used for this purpose, such as use of various linkers (*e.g*., peptide linkers, streptavidin-biotin linkers).

### Molecules coupled to antigen presenting platforms

Molecules coupled to antigen presenting platforms include at least one T cell affecting molecule and at least one antigen presenting complex that comprises at least one antigen binding cleft. Optionally, an antigen can be bound to the antigen binding cleft. These components are discussed below.

### Antigen Presenting Complexes

Antigen presenting complexes comprise an antigen binding cleft and can bind an antigen for presentation to a T cell or T cell precursor. Antigen presenting complexes can be, for example, MHC class I or class II molecules, fusion proteins comprising functional antigen binding clefts of MHC class I or class II molecules, MHC class I or class II "molecular complexes" (described below), or non-classical MHC-like molecules such as members of the CD1 family (*e.g*., CD1a, CD1b, CD1c, CD1d, and CD1e).

In some embodiments, the antigen presenting complexes are MHC class I and/or MHC class II molecular complexes. MHC class I and class II molecular complexes have a number of useful features. For example, they are extremely stable and easy to produce, based on the stability and secretion efficiency provided by the immunoglobulin backbone. Further, by altering the Fc portion of the immunoglobulin, different biological functions can be provided to the molecule based on biological functions afforded by the Fc portion. Substitution of the Fc portion of one type of immunoglobulin gene for another is within the skill of the art.

### MHC class I molecular complexes

"MHC class I molecular complexes" are described in U.S. Patent 6,268,411. MHC class I molecular complexes are formed in a conformationally intact fashion at the ends of the immunoglobulin heavy chains (see FIG. 1A of U.S. Patent 6,268,411 for a schematic representation). MHC class I molecular complexes to which antigenic peptides are bound can stably bind to unique clonotypic lymphocyte receptors (*e.g*., T cell receptors).

MHC class I molecular complexes comprise at least two fusion proteins. A first fusion protein comprises a first MHC class I α chain and a first immunoglobulin heavy chain, and a second fusion protein comprises a second MHC class I α chain and a second immunoglobulin heavy chain. The first and second immunoglobulin heavy chains associate to form the MHC class I molecular complex, which comprises two MHC class I peptide binding clefts. The immunoglobulin heavy chain can be the heavy chain of an IgM, IgD, IgG1, IgG3, IgA2_{β}, IgG2_{α}, IgE, or IgA. Preferably, an IgG heavy chain is used to form MHC class I molecular complexes. If multivalent MHC class I molecular complexes are desired, IgM or IgA heavy chains can be used to provide pentavalent or tetravalent molecules, respectively. MHC class I molecular complexes with other valencies can also be constructed, using multiple immunoglobulin heavy chains. Construction of MHC class I molecular complexes is described in detail in U.S. Patent 6,268,411.

### MHC class II molecular complexes

"MHC class II molecular complexes" are described in U.S. Patent 6,458,354, U.S. Patent 6,015,884, U.S. Patent 6,140,113, and U.S. Patent 6,448,071. MHC class II molecular complexes comprise at least four fusion proteins. Two first fusion proteins comprise (i) an immunoglobulin heavy chain and (ii) an extracellular domain of an MHC class IIβ chain. Two second fusion proteins comprise (i) an immunoglobulin κ or λ light chain and (ii) an extracellular domain of an MHC class IIα chain. The two first and the two second fusion proteins associate to form the MHC class II molecular complex. The extracellular domain of the MHC class IIβ chain of each first fusion protein and the extracellular domain of the MHC class IIα chain of each second fusion protein form an MHC class II peptide binding cleft.

The immunoglobulin heavy chain can be the heavy chain of an IgM, IgD, IgG3, IgG1, IgG2_{β}, IgG2_{α}, IgE, or IgA. Preferably, an IgG1 heavy chain is used to form divalent molecular complexes comprising two antigen binding clefts. Optionally, a variable region of the heavy chain can be included. IgM or IgA heavy chains can be used to provide pentavalent or tetravalent molecular complexes, respectively. Molecular complexes with other valencies can also be constructed, using multiple immunoglobulin chains.

Fusion proteins of an MHC class II molecular complex can comprise a peptide linker inserted between an immunoglobulin chain and an extracellular domain of an MHC class II polypeptide. The length of the linker sequence can vary, depending upon the flexibility required to regulate the degree of antigen binding and receptor cross-linking. Constructs can also be designed such that the extracellular domains MHC class II polypeptides are directly and covalently attached to the immunoglobulin molecules without an additional linker region.

If a linker region is included, this region will preferably contain at least 3 and not more than 30 amino acids. More preferably, the linker is about 5 and not more than 20 amino acids; most preferably, the linker is less than 10 amino acids. Generally, the linker consists of short glycine/serine spacers, but any amino acid can be used. A preferred linker for connecting an immunoglobulin heavy chain to an extracellular domain of an MHC class II β chain is GLY-GLY-GLY-THR-SER-GLY (SEQ ID NO:1). A preferred linker for connecting an immunoglobulin light chain to an extracellular domain of an MHC class IIα chain is GLY-SER-LEU-GLY-GLY-SER (SEQ ID NO:2).

### T cell affecting molecules

T cell affecting molecules are molecules that have a biological effect on a precursor T cell or on an antigen-specific T cell. Such biological effects include, for example, differentiation of a precursor T cell into a CTL, helper T cell (*e*.*g*., Th1, Th2), or regulatory T cell; proliferation of T cells; and induction of T cell apoptosis. Thus, T cell affecting molecules include T cell costimulatory molecules, adhesion molecules, T cell growth factors, regulatory T cell inducer molecules, and apoptosis-inducing molecules. Antigen presenting platforms of the invention comprise at least one such molecule; optionally, an antigen presenting platform comprises at least two, three, or four such molecules, in any combination.

T cell costimulatory molecules contribute to the activation of antigen-specific T cells. Such molecules include, but are not limited to, molecules that specifically bind to CD28 (including antibodies), CD80 (B7-1), CD86 (B7-2), B7-H3, 4-1BBL, CD27, CD30, CD134 (OX-40L), B7h (B7RP-1), CD40, LIGHT, antibodies that specifically bind to HVEM, antibodies that specifically bind to CD40L, antibodies that specifically bind to OX40, and antibodies that specifically bind to 4-1BB.

Adhesion molecules useful for antigen presenting platforms of the invention mediate the adhesion of the platform to a T cell or to a T cell precursor. Adhesion molecules useful in the present invention include, for example, ICAM-1 and LFA-3.

T cell growth factors affect proliferation and/or differentiation of T cells. Examples of T cell growth factors include cytokines (*e.g*., interleukins, interferons) and superantigens. Particularly useful cytokines include IL-2, IL-4, IL-7, IL-10, IL-12, IL-15, and gamma interferon. If desired, cytokines can be present in molecular complexes comprising fusion proteins. In one embodiment, a cytokine molecular complex can comprise at least two fusion proteins: a first fusion protein comprises a first cytokine and an immunoglobulin heavy chain and a second fusion protein comprises a second cytokine and a second immunoglobulin heavy chain. The first and second immunoglobulin heavy chains associate to form the cytokine molecular complex. In another embodiment, a cytokine molecular complex comprises at least four fusion proteins: two first fusion proteins comprise (i) an immunoglobulin heavy chain and (ii) a first cytokine and two second fusion proteins comprise (i) an immunoglobulin light chain and (ii) a second cytokine. The two first and the two second fusion proteins associate to form the cytokine molecular complex. The first and second cytokines in either type of cytokine molecular complex can be the same or different.

Superantigens are the powerful T cell mitogens. Superantigens stimulate T cell mitogenesis by first binding to class II major histocompatibility (MHC) molecules and then as a binary complex bind in a Vβ-specific manner to the T cell antigen receptor (TCR). Superantigens include, but are not limited to, bacterial enterotoxins, such as staphylococcal enterotoxins (*e.g*., SEA and active portions thereof, disclosed in U.S. Patent 5,859,207; SEB, SEC, SED and SEE retroviral superantigens (disclosed in U.S. Patent 5,519,114); *Streptococcus pyogenes* exotoxin (SPE), *Staphylococcus aureus* toxic shock-syndrome toxin (TSST-1), a streptococcal mitogenic exotoxin (SME) and a streptococcal superantigen (SSA) (disclosed in US 2003/0039655); and superantigens disclosed in US 2003/0036644 and US 2003/0009015.

Regulatory T cell inducer molecules are molecules that induce differentiation and/or maintenance of regulatory T cells. Such molecules include, but are not limited to, TGFβ, IL-10, interferon-α, and IL-15. *See, e*.*g*., US 2003/0049696, US 2002/0090724, US 2002/0090357, US 2002/0034500, and US 2003/0064067.

Apoptosis-inducing molecules cause cell death. Apoptosis-inducing molecules include toxins (*e*.*g*., ricin A chain, mutant *Pseudomonas* exotoxins, diphtheria toxoid, streptonigrin, boamycin, saporin, gelonin, and pokeweed antiviral protein), TNFα, and Fas ligand.

### Antigens

A variety of antigens can be bound to antigen presenting complexes. The nature of the antigens depends on the type of antigen presenting complex that is used. For example, peptide antigens can be bound to MHC class I and class II peptide binding clefts. Non-classical MHC-like molecules can be used to present non-peptide antigens such as phospholipids, complex carbohydrates, and the like (*e.g*., bacterial membrane components such as mycolic acid and lipoarabinomannan). "Antigens" as used herein also includes "antigenic peptides."

### Antigenic Peptides

Any peptide capable of inducing an immune response can be bound to an antigen presenting complex. Antigenic peptides include tumor-associated antigens, autoantigens, alloantigens, and antigens of infectious agents.

### Tumor-Associated Antigens

Tumor-associated antigens include unique tumor antigens expressed exclusively by the tumor from which they are derived, shared tumor antigens expressed in many tumors but not in normal adult tissues (oncofetal antigens), and tissue-specific antigens expressed also by the normal tissue from which the tumor arose. Tumor-associated antigens can be, for example, embryonic antigens, antigens with abnormal post-translational modifications, differentiation antigens, products of mutated oncogenes or tumor suppressors, fusion proteins, or oncoviral proteins.

A variety of tumor-associated antigens are known in the art, and many of these are commercially available. Oncofetal and embryonic antigens include carcinoembryonic antigen and alpha-fetoprotein (usually only highly expressed in developing embryos but frequently highly expressed by tumors of the liver and colon, respectively), MAGE-1 and MAGE-3 (expressed in melanoma, breast cancer, and glioma), placental alkaline phosphatase sialyl-Lewis X (expressed in adenocarcinoma), CA-125 and CA-19 (expressed in gastrointestinal, hepatic, and gynecological tumors), TAG-72 (expressed in colorectal tumors), epithelial glycoprotein 2 (expressed in many carcinomas), pancreatic oncofetal antigen, 5T4 (expressed in gastric carcinoma), alphafetoprotein receptor (expressed in multiple tumor types, particularly mammary tumors), and M2A (expressed in germ cell neoplasia).

Tumor-associated differentiation antigens include tyrosinase (expressed in melanoma) and particular surface immunoglobulins (expressed in lymphomas).

Mutated oncogene or tumor-suppressor gene products include Ras and p53, both of which are expressed in many tumor types, Her-2/neu (expressed in breast and gynecological cancers), EGF-R, estrogen receptor, progesterone receptor, retinoblastoma gene product, myc (associated with lung cancer), ras, p53, nonmutant associated with breast tumors, MAGE-1, and MAGE-3 (associated with melanoma, lung, and other cancers).

Fusion proteins include BCR-ABL, which is expressed in chromic myeloid leukemia.

Oncoviral proteins include HPV type 16, E6, and E7, which are found in cervical carcinoma.

Tissue-specific antigens include melanotransferrin and MUC1 (expressed in pancreatic and breast cancers); CD10 (previously known as common acute lymphoblastic leukemia antigen, or CALLA) or surface immunoglobulin (expressed in B cell leukemias and lymphomas); the α chain of the IL-2 receptor, T cell receptor, CD45R, CD4⁺/CD8⁺ (expressed in T cell leukemias and lymphomas); prostate-specific antigen and prostatic acid-phosphatase (expressed in prostate carcinoma); GP 100, MelanA/Mart-1, tyrosinase, gp75/brown, BAGE, and S-100 (expressed in melanoma); cytokeratins (expressed in various carcinomas); and CD19, CD20, and CD37 (expressed in lymphoma).

Tumor-associated antigens also include altered glycolipid and glycoprotein antigens, such as neuraminic acid-containing glycosphingolipids (*e*.*g*., GM₂ and GD₂, expressed in melanomas and some brain tumors); blood group antigens, particularly T and sialylated Tn antigens, which can be aberrantly expressed in carcinomas; and mucins, such as CA-125 and CA-19-9 (expressed on ovarian carcinomas) or the underglycosylated MUC-1 (expressed on breast and pancreatic carcinomas).

Tissue-specific antigens include epithelial membrane antigen (expressed in multiple epithelial carcinomas), CYFRA 21-1 (expressed in lung cancer), Ep-CAM (expressed in pan-carcinoma), CA125 (expressed in ovarian cancer), intact monoclonal immunoglobulin or light chain fragments (expressed in myeloma), and the beta subunit of human chorionic gonadotropin (HCG, expressed in germ cell tumors).

### Autoantigens

An autoantigen is an organism's own "self antigen" to which the organism produces an immune response. Autoantigens are involved in autoimmune diseases such as Goodpasture's syndrome, multiple sclerosis, Graves' disease, myasthenia gravis, systemic lupus erythematosus, insulin-dependent diabetes mellitis, rheumatoid arthritis, pemphigus vulgaris, Addison's disease, dermatitis herpetiformis, celiac disease, and Hashimoto's thyroiditis.

Diabetes-related autoantigens include insulin, glutamic acid decarboxylase (GAD) and other islet cell autoantigens, *e.g*., ICA 512/IA-2 protein tyrosine phosphatase, ICA12, ICA69, preproinsulin or an immunologically active fragment thereof (*e.g*., insulin B-chain, A chain, C peptide or an immunologically active fragment thereof), HSP60, carboxypeptidase H, peripherin, gangliosides (*e.g*., GM1-2, GM3) or immunologically active fragments thereof.

Macular degeneration-associated autoantigens include complement pathway molecules and various autoantigens from RPE, choroid, and retina, vitronectin, β crystallin, calreticulin, serotransferrin, keratin, pyruvate carboxylase, C1, and villin 2.

Other autoantigens include nucleosomes (particles containing histones and DNA); ribonucleoprotein (RNP) particles (containing RNA and proteins that mediate specialized functions in the RNP particle), and double stranded DNA. Still other autoantigens include myelin oligodendrocyte glycoprotein (MOG), myelin associated glycoprotein (MAG), myelin/oligodendrocyte basic protein (MOBP), Oligodendrocyte specific protein (Osp), myelin basic protein (MBP), proteolipid apoprotein (PLP), galactose cerebroside (GalC), glycolipids, sphingolipids, phospholipids, gangliosides and other neuronal antigens.

### Alloantigens

An alloantigen is a direct or indirect product of an allele that is detected as an antigen by another member of the same species. Direct products of such alleles include encoded polypeptides; indirect products include polysaccharides and lipids synthesized by allele-encoded enzymes. Alloantigens include major and minor histocompatibility antigens (known as HLA in humans), including class I and class II antigens, blood group antigens such as the ABO, Lewis group, antigens on T and B cells, and monocyte/endothelial cell antigens. HLA specificities include A (*e.g*. A1-A74, particularly A1, A2, A3, A11, A23, A24, A28, A30, A33), B (*e.g*., B1-B77, particularly B7, B8, B35, B44, B53, B60, B62), C (*e.g*., C1-C11), D (*e.g*., D1-D26), DR (*e.g*., DR1, DR2, DR3, DR4, DR7, DR8, and DR 11), DQ (*e.g.,* DQ1-DQ9), and DP (*e.g.,* DP1-DP6).

### Antigens of infectious agents

Antigens of infectious agents include components of protozoa, bacteria, fungi (both unicellular and multicellular), viruses, prions, intracellular parasites, helminths, and other infectious agents that can induce an immune response.

Bacterial antigens include antigens of gram-positive cocci, gram positive bacilli, gram-negative bacteria, anaerobic bacteria, such as organisms of the families *Actinomycetaceae, Bacillaceae, Bartonellaceae, Bordetellae, Captophagaceae, Corynebacteriaceae, Enterobacteriaceae, Legionellaceae, Micrococcaceae, Mycobacteriaceae, Nocardiaceae, Pasteurellaceae, Pseudomonadaceae, Spirochaetaceae, Vibrionaceae* and organisms of the genera *Acinetobacter, Brucella, Campylobacter, Erysipelothrix, Ewingella, Francisella, Gardnerella, Helicobacter, Levinea, Listeria, Streptobacillus and Tropheryma.*

Antigens of protozoan infectious agents include antigens of malarial plasmodia, *Leishmania* species, *Trypanosoma* species and *Schistosoma* species.

Fungal antigens include antigens of *Aspergillus, Blastomyces, Candida, Coccidioides, Cryptococcus, Histoplasma, Paracoccicioides, Sporothrix,* organisms of the order *Mucorales,* organisms inducing choromycosis and mycetoma and organisms of the genera *Trichophyton, Microsporum, Epidermophyton,* and *Malassezia*.

Antigens of prions include the sialoglycoprotein PrP 27-30 of the prions that cause scrapie, bovine spongiform encephalopathies (BSE), feline spongiform encephalopathies, kuru, Creutzfeldt-Jakob Disease (CJD), Gerstmann-Strassler-Scheinker Disease (GSS), and fatal familial insomnia (FFI).

Intracellular parasites from which antigenic peptides can be obtained include, but are not limited to, *Chlamydiaceae, Mycoplasmataceae, Acholeplasmataceae, Rickettsiae,* and organisms of the genera *Coxiella* and *Ehrlichia*.

Antigenic peptides can be obtained from helminths, such as nematodes, trematodes, or cestodes.

Viral peptide antigens include, but are not limited to, those of adenovirus, herpes simplex virus, papilloma virus, respiratory syncytial virus, poxviruses, HIV, influenza viruses, and CMV. Particularly useful viral peptide antigens include HIV proteins such as HIV gag proteins (including, but not limited to, membrane anchoring (MA) protein, core capsid (CA) protein and nucleocapsid (NC) protein), HIV polymerase, influenza virus matrix (M) protein and influenza virus nucleocapsid (NP) protein, hepatitis B surface antigen (HBsAg), hepatitis B core protein (HBcAg), hepatitis e protein (HBeAg), hepatitis B DNA polymerase, hepatitis C antigens, and the like.

### Binding antigens to antigen presenting complexes

Antigens, including antigenic peptides, can be bound to an antigen binding cleft of an antigen presenting complex either actively or passively, as described in U.S. Patent 6,268,411. Optionally, an antigenic peptide can be covalently bound to a peptide binding cleft.

If desired, a peptide tether can be used to link an antigenic peptide to a peptide binding cleft. For example, crystallographic analyses of multiple class I MHC molecules indicate that the amino terminus of β2M is very close, approximately 20.5 Angstroms away, from the carboxyl terminus of an antigenic peptide resident in the MHC peptide binding cleft. Thus, using a relatively short linker sequence, approximately 13 amino acids in length, one can tether a peptide to the amino terminus of β2M. If the sequence is appropriate, that peptide will bind to the MHC binding groove (see U.S. Patent 6,268,411).

### Molecules coupled to antibody inducing platforms

Molecules coupled to antibody inducing platforms include at least one B cell affecting molecule and at least one molecular complex that can engage B cell surface immunoglobulins or that can engage antigen-containing MHC complexes on the surface of a B cell.

### B cell affecting molecules

B cell affecting molecules are molecules that have a biological effect on a B cell or a B cell precursor, such as inducing proliferation or antibody formation. Such molecules include CD40 ligand, as well as cytokines and cytokine molecular complexes as described above. Depending on the type of cytokine molecule used, B cells can be encouraged to produce particular types of antibodies. For example, IL-4 induces the production of IgE, whereas IL-5 induces the production of IgA.

### Molecular complexes

Molecular complexes for use on antibody inducing platforms are complexes that engage B cell surface immunoglobulins or that engage MHC-antigen complexes on the surface of a B cell. Molecular complexes that engage B cell surface immunoglobulins include antigens complexed to the platform surface. Molecular complexes that engage MHC-antigen complexes on the surface of a B cell include T cell receptors (TCRs) and TCR molecular complexes. Antibody inducing platforms can include one or both forms (*i*.*e*., B cell surface immunoglobulin engaging or MHC-antigen engaging) of such molecular complexes.

TCRs specific for any particular antigen can be cloned using methods well known in the art. *See, e.g.,* US 2002/0064521. Cloned antigen-specific TCRs can be used as such or can be used to form TCR molecular complexes, described below.

### TCR molecular complexes

"TCR molecular complexes" are disclosed in U.S. Patent 6,458,354, U.S. Patent 6,015,884, U.S. Patent 6,140,113, and U.S. Patent 6,448,071. TCR molecular complexes comprise at least four fusion proteins. Two first fusion proteins comprise (i) an immunoglobulin heavy chain and (ii) an extracellular domain of a TCR α chain. Two second fusion proteins comprise (i) an immunoglobulin κ or λ light chain and (ii) an extracellular domain of TCR β chain. Alternatively, two first fusion proteins comprise (i) an immunoglobulin heavy chain and (ii) an extracellular domain of a TCR γ chain, and two second fusion proteins comprise (i) an immunoglobulin κ or λ light chain and (ii) an extracellular domain of TCR δ chain. The two first and the two second fusion proteins associate to form the TCR molecular complex. The extracellular domain of the TCR chain of each first fusion protein and the extracellular domain of the TCR chain of each second fusion protein form an antigen recognition cleft.

The immunoglobulin heavy chain can be the heavy chain of an IgM, IgD, IgG3, IgG1, IgG2_{β}, IgG2_{α},, IgE, or IgA. Preferably, an IgG1 heavy chain is used to form divalent TCR molecular complexes comprising two antigen recognition clefts. Optionally, a variable region of the heavy chain can be included. IgM or IgA heavy chains can be used to provide pentavalent or tetravalent TCR molecular complexes, respectively. TCR molecular complexes with other valencies can also be constructed, using multiple immunoglobulin chains.

Fusion proteins of a TCR molecular complex can comprise a peptide linker inserted between an immunoglobulin chain and an extracellular domain of a TCR polypeptide. The length of the linker sequence can vary, depending upon the flexibility required to regulate the degree of antigen binding and cross-linking. Constructs can also be designed such that the extracellular domains of TCR polypeptides are directly and covalently attached to the immunoglobulin molecules without an additional linker region. If a linker region is included, this region will preferably contain at least 3 and not more than 30 amino acids. More preferably, the linker is about 5 and not more than 20 amino acids; most preferably, the linker is less than 10 amino acids. Generally, the linker consists of short glycine/serine spacers, but any amino acid can be used. A preferred linker for connecting an immunoglobulin heavy chain to an extracellular domain of a TCR α or γ chain is GLY-GLY-GLY-THR-SER-GLY (SEQ ID NO:1). A preferred linker for connecting an immunoglobulin light chain to an extracellular domain of a TCR β or δ chain is GLY-SER-LEU-GLY-GLY-SER (SEQ ID NO:2).

### Methods of using platforms of the invention to induce and expand specific cell populations

### Induction and expansion of antigen-specific T cells

The invention provides methods of inducing the formation and expansion of antigen-specific T cells, including CTLs, helper T cells, and regulatory T cells. These methods involve contacting an isolated preparation comprising a plurality of precursor T cells with antigen presenting platforms of the invention to which antigens are bound to the antigenic binding clefts. Incubation of the preparation with the antigen presenting platforms induces precursor cells in the population to form antigen-specific T cells that recognize the antigen. Antigen-specific T cells can be obtained by incubating precursor T cells with antigen presenting platforms of the invention, as described below, or can be obtained by conventional methods, *e.g*., incubation with dendritic cells, or by incubating with other types of artificial antigen presenting cells as are known in the art.

Typically, either the number or the percentage of antigen-specific T cells in the first cell population is greater than the number or percentage of antigen-specific T cells that are formed if precursor T cells are incubated with particles that comprise an antibody that specifically binds to CD3 but do not comprise an antigen presenting complex.

In any of the embodiments disclosed herein in which antigen presenting platforms are used, any combination of antigen presenting complexes, bound antigens, and T cell affecting molecules can be used. For example, an antigen presenting platform can comprise one or more T cell costimulatory molecules (either the same or different), one or more regulatory T cell inducing molecules (either the same or different), one or more adhesion molecules (either the same or different), and/or one or more T cell growth factors (either the same or different). Similarly, any particular antigen presenting platform can comprise one or more antigen presenting complexes, either the same or different, to which any combination of antigens can be bound. In one embodiment, for example, several different melanoma-associated antigens (*e.g*., any or all of tyrosinase, MAGE-1, MAGE-3, GP-100, Melan A/Mart-1, gp75/brown, BAGE, and S-100) can be bound to antigen presenting complexes on one or more platforms.

Precursor T cells can be obtained from the patient or from a suitable donor. The donor need not be an identical twin or even related to the patient. Preferably, however, the donor and the patient share at least one HLA molecule. Precursor T cells can be obtained from a number of sources, including peripheral blood mononuclear cells, bone marrow, lymph node tissue, spleen tissue, and tumors. Alternatively, T cell lines available in the art can be used.

In one embodiment, precursor T cells are obtained from a unit of blood collected from a subject using any number of techniques known to the skilled artisan, such as Ficoll separation. For example, precursor T cells from the circulating blood of an individual can be obtained by apheresis or leukapheresis. The apheresis product typically contains lymphocytes, including T cells and precursor T cells, monocytes, granulocytes, B cells, other nucleated white blood cells, red blood cells, and platelets. Cells collected by apheresis can be washed to remove the plasma fraction and to place the cells in an appropriate buffer or media for subsequent processing steps. Washing steps can be accomplished by methods known to those in the art, such as by using a semi-automated "flow-through" centrifuge (for example, the Cobe 2991 cell processor) according to the manufacturer's instructions. After washing, the cells may be resuspended in a variety of biocompatible buffers, such as, for example, Ca-free, Mg-free PBS. Alternatively, the undesirable components of the apheresis sample can be removed and the cells directly resuspended in a culture medium. If desired, precursor T cells can be isolated from peripheral blood lymphocytes by lysing the red blood cells and depleting the monocytes, for example, by centrifugation through a PERCOLL™ gradient.

Optionally, a cell population comprising antigen-specific T cells can continue to be incubated with either the same antigen presenting platform or a second antigen presenting platform for a period of time sufficient to form a second cell population comprising an increased number of antigen-specific T cells relative to the number of antigen-specific T cells in the first cell population. Typically, such incubations are carried out for 3-21 days, preferably 7-10 days.

Suitable incubation conditions (culture medium, temperature, etc.) include those used to culture T cells or T cell precursors, as well as those known in the art for inducing formation of antigen-specific T cells using DC or artificial antigen presenting cells. *See*, *e.g*., Latouche & Sadelain, Nature Biotechnol. 18, 405-09, April 2000; Levine et al., J. Immunol. 159, 5921-30, 1997; Maus et al., Nature Biotechnol. 20, 143-48, February 2002. *See also* the specific examples, below.

### Optimizing the duration of interaction between antigen presenting platforms and T cells

One difference between T cell stimulation by some antigen presenting platforms of the invention and that by ordinary normal dendritic cells is the duration of stimulation required. For example, recognition of a normal DC by CTLs ultimately leads to lysis and elimination of antigenic stimulus by the activated T cell. In contrast, T cells may not have an effective way of eliminating antigen on an antigen presenting platform, particularly one based on an artificial, non-biodegradable surface. Thus, stimulation by the platform could potentially go on for hours if not days.

To assess the magnitude of a proliferative signal, antigen-specific T cell populations can be labeled with CFSE and analyzed for the rate and number of cell divisions. T cells can be labeled with CFSE after one-two rounds of stimulation with either antigen presenting platforms of the invention to which an antigen is bound. At that point, antigen-specific T cells should represent 2-10% of the total cell population. The antigen-specific T cells can be detected using antigen-specific staining so that the rate and number of divisions of antigen-specific T cells can be followed by CFSE loss. At varying times (for example, 12, 24, 36, 48, and 72 hours) after stimulation, the cells can be analyzed for both antigen presenting complex staining and CFSE. Stimulation with antigen presenting platforms to which an antigen has not been bound can be used to determine baseline levels of proliferation. Optionally, proliferation can be detected by monitoring incorporation of ³H-thymidine, as is known in the art.

Cultures can stimulated for variable amounts of time (*e.g*., 0.5, 2, 6, 12, 36 hours as well as continuous stimulation) with antigen presenting platforms of the invention. Particle- or cell-based platforms can be separated from T cells by vigorous pipetting to disrupt any conjugates. Artificial particle-based platforms can be isolated by gravity; cell-based platforms can be isolated, *e.g*., using FACS. The effect of stimulation time in highly enriched antigen-specific T cell cultures can be assessed, and conditions can be identified under which a large percentage (*e.g*., 50, 70, 75, 80, 85, 90, 95, or 98%) of platforms can be recovered with little cell loss. Antigen-specific T cell can then be placed back in culture and analyzed for cell growth, proliferation rates, effects on apoptosis, various effector functions, and the like, as is known in the art. Such conditions may vary depending on the antigen-specific T cell response desired.

### Detection of antigen-specific T cells

The effect of antigen presenting platforms of the invention on expansion, activation and differentiation of T cell precursors can be assayed in any number of ways known to those of skill in the art. A rapid determination of function can be achieved using a proliferation assay, by determining the increase of CTL, helper T cells, or regulatory T cells in a culture by detecting markers specific to each type of T cell. Such markers are known in the art. CTL can be detected by assaying for cytokine production or for cytolytic activity using chromium release assays.

### Analysis of homing receptors on platform-induced/expanded antigen-specific T cells

In addition to generating antigen-specific T cells with appropriate effector functions, another parameter for antigen-specific T cell efficacy is expression of homing receptors that allow the T cells to traffic to sites of pathology (Sallusto et al., Nature 401, 708-12, 1999; Lanzavecchia & Sallusto, Science 290, 92-97, 2000). The absence of appropriate homing receptors has been implicated in the setting of chronic CMV and EBV infection (Chen et al., Blood 98, 156-64, 2001). In addition, one difference noted between the use of professional APC and nonprofessional APC to expand antigen-specific T cells is expression of appropriate homing receptors, which may account for the presence of *in vivo* dysfunctional CTL (Salio et al., J. Immunol. 167, 1188-97, 2001).

For example, effector CTL efficacy has been linked to the following phenotype of homing receptors, CD62L+, CD45RO+, and CCR7-. Thus, a platform-induced and/or expanded CTL population can be characterized for expression of these homing receptors. Homing receptor expression is a complex trait linked to initial stimulation conditions. Presumably, this is controlled both by the co-stimulatory complexes as well as cytokine milieu. One important cytokine that has been implicated is IL-12 (Salio *et al.,* 2001). As discussed below, platforms of the invention offer the potential to vary individually separate components (*e.g*., T cell effector molecules and antigen presenting complexes) to optimize biological outcome parameters. Optionally, cytokines such as IL-12 can be included in the initial induction cultures to affect homing receptor profiles in an antigen-specific T cell population.

### Analysis of off-rate in induced and/or expanded antigen-specific T cell populations

Evolution of secondary immune responses are associated with focusing of the affinities, as determined by analysis of TCR "off-rates" (Savage et al., Immunity 10, 485-92, 1999; Busch et al., J. Exp. Med. 188, 61-70, 1998; Busch & Pamer, J. Exp. Med. 189, 701-09, 1999). A decrease in TCR-off rates (*i.e*., resulting in increased TCR affinity) is a parameter that correlates well with increased ability to recognize low amounts of antigen and biological efficacy of a T cell population of interest. Off-rates can be optimized by varying the magnitude and/or duration of antigen presenting platform-mediated stimulation.

### Separation of antigen-specific T cells from other cells

Antigen-specific T cells which are bound to antigens can be separated from cells which are not bound. Any method known in the art can be used to achieve this separation, including plasmapheresis, flow cytometry, or differential centrifugation. In one embodiment T cells are isolated by incubation with beads, for example, anti-CD3/anti-CD28-conjugated beads, such as DYNABEADS^{®} M-450 CD3/CD28 T, for a time period sufficient for positive selection of the desired T cells.

If desired, subpopulations of antigen-specific T cells can be separated from other cells that may be present. For example, specific subpopulations of T cells, such as CD28⁺, CD4⁺, CD8⁺, CD45RA⁺, and CD45RO⁺T cells, can be further isolated by positive or negative selection techniques. One method is cell sorting and/or selection via negative magnetic immunoadherence or flow cytometry that uses a cocktail of monoclonal antibodies directed to cell surface markers present on the cells negatively selected. For example, to enrich for CD4⁺ cells by negative selection, a monoclonal antibody cocktail typically includes antibodies to CD14, CD20, CD11b, CD16, HLA-DR, and CD8. Antigen-specific regulatory T cells can be detected and/or separated from other cells using the marker Foxp3. The time period can range from 30 minutes to 36 hours or 10 to 24 hours or can be at least 1, 2, 3, 4, 5, or 6 hours or at least 24 hours. Longer incubation times can be used to isolate T cells in any situation where there are few T cells as compared to other cell types, such in isolating tumor infiltrating lymphocytes (TIL) from tumor tissue or from immunocompromised individuals.

### Induction and expansion of antibody-producing B cells

The invention also provides methods of inducing the formation of antibody-producing B cells. These methods involve contacting an isolated preparation comprising a plurality of precursor B cells with antibody inducing platforms of the invention. Incubation of the preparation with the antibody inducing platforms induces precursor cells in the population to form antibody producing B cells that produce antibodies that specifically recognize the antigen. Typically, either the number or the percentage of antibody-producing B cells in the first cell population is greater than the number or percentage of antibody-producing cells that are formed if precursor B cells are incubated with a non-specific stimulus, *e.g*., phytohemagglutinin (PHA), lipopolysaccharide (LPS), or pokeweed. In any of the embodiments disclosed herein in which antibody inducing platforms are used, any combination of B cell affecting molecules and complexes that engage B cell surface immunoglobulins or MHC-antigen complexes on a B cell surface can be used.

Precursor B cells can be obtained from the patient or from a suitable donor. The donor and the patient need not be related, but preferably share at least one HLA molecule. Alternatively, B cell lines available in the art can be used. In one embodiment, precursor B cells are obtained from a unit of blood collected from a subject using any number of techniques known to the skilled artisan, such as Ficoll separation. For example, precursor B cells from the circulating blood of an individual can be obtained by apheresis or leukapheresis, as discussed above.

B cells or their precursors can be cultured using methods known in the art. *See, e.g.,* Schultze et al., J. Clin. Invest. 100, 2757-65, 1997; von Bergwelt-Baildon et al., Blood 99, 3319-25, 2002. Such conditions also are suitable for incubating B cell precursors with antibody inducing platforms of the invention.

Optionally, a cell population comprising antibody-producing B cells can continue to be incubated with either the same antibody inducing platform or a second antibody inducing platform for a period of time sufficient to form a second cell population comprising an increased number of antibody-producing B cells relative to the number of antibody-producing B cells in the first cell population. Typically, such incubations are carried out for 3-21 days, preferably 7-10 days.

### Optimizing the duration of interaction between antibody inducing platforms and B cells

As with T cells stimulation discussed above, the duration of stimulation required to induce or expand populations of antibody-producing B cells may differ from that occurring normally, particularly if an artificial, non-biodegradable surface is used for the platform. Thus, stimulation by the platform could potentially go on for hours if not days. The duration of interaction between various antibody inducing platforms of the invention and precursor or antibody-producing B cells can be determined using methods similar to those discussed above for antigen-specific T cells.

### Detection of antibody-producing B cells

The effect of antibody-producing platforms of the invention on expansion, activation and differentiation of B cell precursors can be assayed in any number of ways known to those of skill in the art. A rapid determination of function can be achieved using a proliferation assay, by detecting B cell-specific markers, or by assaying for specific antibody production.

### Pharmaceutical preparations

Pharmaceutical preparations comprising particle- or cell-based antigen presenting platforms or antibody inducing platforms of the invention, as well as antigen-specific T cells or antibody-specific B cells obtained using such platforms, can be formulated for direct injection into patients. Such pharmaceutical preparations contain a pharmaceutically acceptable carrier suitable for delivering the compositions of the invention to a patient, such as saline, buffered saline (*e.g*., phosphate buffered saline), or phosphate buffered saline glucose solution.

### Immunotherapeutic methods

### Routes of administration

Particle- or cell-based antigen presenting platforms or antibody inducing platforms of the invention, as well as antigen-specific T cells or antibody-specific B cells obtained using such platforms, can be administered to patients by any appropriate routes, including intravenous administration, intra-arterial administration, subcutaneous administration, intradermal administration, intralymphatic administration, and intra-tumoral administration. Patients include both human and veterinary patients.

### Therapeutic methods

Platforms of the invention can be used to generate therapeutically useful numbers of antigen-specific T cells or antibody-producing B cells that can be used in diagnostic and therapeutic methods known in the art. See, e.g., WO 01/94944; US 2002/0004041; U.S. Patent 5,583,031; US 2002/0119121; US 2002/0122818; U.S. Patent 5,635,363; US 2002/0090357; U.S. Patent 6,458,354; US 2002/0034500.

In particular, antigen-specific T cells or antibody-producing B cells can be used to treat patients with infectious diseases, cancer, or autoimmune diseases, or to provide prophylactic protection to immunosuppressed patients.

Infectious diseases that can be treated include those caused by bacteria, viruses, prions, fungi, parasites, helminths, etc. Such diseases include AIDS, hepatitis, CMV infection, and post-transplant lymphoproliferative disorder (PTLD). CMV, for example, is the most common viral pathogen found in organ transplant patients and is a major cause of morbidity and mortality in patients undergoing bone marrow or peripheral blood stem cell transplants (Zaia, Hematol. Oncol. Clin. North Am. 4, 603-23, 1990). This is due to the immunocompromised status of these patients, which permits reactivation of latent virus in seropositive patients or opportunistic infection in seronegative individuals. Current treatment focuses on the use of antiviral compounds such as gancyclovir, which have drawbacks, the most significant being the development of drug-resistant CMV. A useful alternative to these treatments is a prophylactic immunotherapeutic regimen involving the generation of virus-specific CTL derived from the patient or from an appropriate donor before initiation of the transplant procedure.

PTLD occurs in a significant fraction of transplant patients and results from Epstein-Barr virus (EBV) infection. EBV infection is believed to be present in approximately 90% of the adult population in the United States (Anagnostopoulos & Hummel, Histopathology 29, 297-315, 1996). Active viral replication and infection is kept in check by the immune system, but, as in cases of CMV, individuals immunocompromised by transplantation therapies lose the controlling T cell populations, which permits viral reactivation. This represents a serious impediment to transplant protocols. EBV may also be involved in tumor promotion in a variety of hematological and non-hematological cancers. There is also a strong association between EBV and nasopharyngeal carcinomas. Thus a prophylactic treatment with EBV-specific T cells offers an excellent alternative to current therapies.

Cancers that can be treated according to the invention include melanoma, carcinomas, *e.g*., colon, duodenal, prostate, breast, ovarian, ductal, hepatic, pancreatic, renal, endometrial, stomach, dysplastic oral mucosa, polyposis, invasive oral cancer, non-small cell lung carcinoma, transitional and squamous cell urinary carcinoma etc.; neurological malignancies, *e*.*g*., neuroblastoma, gliomas, etc.; hematological malignancies, *e*.*g*., chronic myelogenous leukemia, childhood acute leukemia, non-Hodgkin's lymphomas, chronic lymphocytic leukemia, malignant cutaneous T-cells, mycosis fungoides, non-MF cutaneous T-cell lymphoma, lymphomatoid papulosis, T-cell rich cutaneous lymphoid hyperplasia, bullous pemphigoid, discoid lupus erythematosus, lichen planus, etc.; and the like.. *See, e.g.,* Mackensen et al., Int. J. Cancer 86, 385-92, 2000; Jonuleit et al., Int. J. Cancer 93, 243-51, 2001; Lan et al., J. Immunotherapy 24, 66-78, 2001; Meidenbauer et al., J. Immunol. 170(4), 2161-69, 2003.

Autoimmune diseases that can be treated include asthma, systemic lupus erythematosus, rheumatoid arthritis, type I diabetes, multiple sclerosis, Crohn's disease, ulcerative colitis, psoriasis, myasthenia gravis, Goodpasture's syndrome, Graves' disease, pemphigus vulgaris, Addison's disease, dermatitis herpetiformis, celiac disease, and Hashimoto's thyroiditis.

Antigen-specific helper T cells can be used to activate macrophages or to activate B cells to produce specific antibodies that can be used, for example, to treat infectious diseases and cancer. Antibody-producing B cells themselves also can be used for this purpose.

Antigen-specific regulatory T cells can be used to achieve an immunosuppressive effect, for example, to treat or prevent graft versus host disease in transplant patients, or to treat or prevent autoimmune diseases, such as those listed above, or allergies. Uses of regulatory T cells are disclosed, for example, in US 2003/0049696, US 2002/0090724, US 2002/0090357, US 2002/0034500, and US 2003/0064067. Antigen presenting platforms in which the T cell affecting molecule is an apoptosis-inducing molecule can be used to suppress immune responses.

### Doses

Antigen-specific T cells can be administered to patients in doses ranging from about 5-10 x 10⁶ CTL/kg of body weight (∼7 x10⁸ CTL/treatment) up to about 3.3 x 10⁹ CTL/m² (∼6 x 10⁹ CTL/treatment) (Walter et al., New England Journal of Medicine 333, 1038-44, 1995; Yee et al., J Exp Med 192, 1637-44, 2000). In other embodiments, patients can receive 10³, 5 x 10³, 10⁴, 5 x 10⁴, 10⁵, 5 x 10⁵, 10⁶, 5 x 10⁶, 10⁷, 5 x 10⁷, 10⁸, 5 x 10⁸, 10⁹, 5 x 10⁹, or 10¹⁰ cells per dose administered intravenously. In still other embodiments, patients can receive intranodal injections of, *e.g*., 8 x 10⁶ or 12 x 10⁶ cells in a 200 µl bolus. Cell-based antigen presenting platforms or antibody inducing platforms, as well as antibody-producing B cells themselves, can be administered to patients in similar doses.

If particle-based platforms are administered, typical doses include 10³, 5 x 10³, 10⁴, 5 x 10⁴, 10⁵, 5 x 10⁵, 10⁶, 5 x 10⁶, 10⁷, 5 x 10⁷, 10⁸, 5 x 10⁸, 10⁹, 5 x 10⁹, or 10¹⁰ particles per dose.

### Animal Models

A number of murine models are available to assess adoptive immunotherapy protocols for tumor treatment. Two models are particularly suitable for assessing melanoma treatment. One model uses human/SCID mice bearing a subcutaneous implanted human melanoma line, such as BML. In such models, transfer of *ex vivo* expanded Mart-1-specific CTL delays the onset and/or growth of the tumor. A second model uses the murine A2-transgenic mice and the murine B16 melanoma that has been transfected with an HLA-A2-like molecule, called AAD. This molecule, which is also the basis of the A2-transgenic, is human HLA-A2 in alpha 1-2 domains fused to the murine alpha3 domain. Using these transgenic mice, the murine B16-AAD melanoma is sensitive to rejection across well-defined A2-resticted melanoma epitopes derived from tyrosinase and gp100.

### Kits

Either antigen presenting platforms or antibody inducing platforms of the invention can be provided in kits. Suitable containers for particle- or cell-based antigen presenting or antibody inducing platforms include, for example, bottles, vials, syringes, and test tubes. Containers can be formed from a variety of materials, including glass or plastic. A container may have a sterile access port (for example, the container may be an intravenous solution bag or a vial having a stopper pierceable by a hypodermic injection needle). Alternatively, kits can comprise a rigid or flexible antigen presenting or antibody inducing platform, as described above. Optionally, one or more different antigens can be bound to the platforms or can be supplied separately.

A kit can further comprise a second container comprising a pharmaceutically-acceptable buffer, such as phosphate-buffered saline, Ringer's solution, or dextrose solution. It can also contain other materials useful to an end user, including other buffers, diluents, filters, needles, and syringes. A kit can also comprise a second or third container with another active agent, for example a chemotherapeutic agent or an anti-infective agent, or containing particular antigens that can be bound to antigen presenting complexes of an antigen presenting platform by an end user.

Kits also can contain reagents for assessing the extent and efficacy of antigen-specific T cell or antibody-producing B cell induction or expansion, such as antibodies against specific marker proteins, MHC class I or class II molecular complexes, TCR molecular complexes, anticlonotypic antibodies, and the like.

A kit can also comprise a package insert containing written instructions for methods of inducing antigen-specific T cells, expanding antigen-specific T cells, using antigen presenting platforms or antibody inducing platforms in the kit in various treatment protocols. The package insert can be an unapproved draft package insert or can be a package insert approved by the Food and Drug Administration (FDA) or other regulatory body.

The above disclosure generally describes the present invention. A more complete understanding can be obtained by reference to the following specific examples, which are provided for purposes of illustration only and are not intended to limit the scope of the invention.

### EXAMPLE 1

### Materials and Methods

*Cell Lines*. TAP-deficient 174CEM.T2 (T2) cells and melanoma cell lines were maintained in M' medium (Oelke et al., Scand. J Immunol. 52, 544-49, 2000) supplemented with 10% FCS.

*Peptides*. Peptides (Mart-1, ELAGIGILTV, SEQ ID NO:3; CMVpp65, NLVPMVATV, SEQ ID NO:4) used in this study were prepared by the JHU core facility. The purity (>98%) of each peptide was confirmed by mass-spectral analysis and HPLC.

*HLA-A2.1*+ *Lymphocytes*. The Institutional Ethics Committee at The Johns Hopkins University approved the studies discussed in the examples below. All donors gave written informed consent before enrolling in the study. Healthy volunteers and a melanoma patient, donor #7, were phenotyped HLA-A2.1 by flow cytometry. The melanoma patient had extensive metastatic disease with lung, liver, and lymph node metastases. PBMC were isolated by Ficoll-Hypaque density gradient centrifugation.

*Generation of aAPC.* aAPC were generated by coupling "HLA-Ig" (described in U.S. Patent 6,268,411) and anti-CD28 onto microbeads (Dynal, Lake Success, NY). Briefly, beads were washed twice in sterile 0.1 M borate buffer ("bead wash buffer"). The beads were incubated with a 1 to1 mixture of the HLA-A2-Ig and the anti-CD28 mAb 9.3 in borate buffer for 24 h at 4°C on a rotator and washed twice with bead wash buffer. After another 24 h incubation at 4°C in bead wash buffer, the buffer was replaced. Resulting aAPC were found to have 0.9 x 10⁵ molecules of A2-Ig/bead and 1.9 x 10⁵ anti-CD28 molecules/bead. aAPC beads were stored at 4°C for longer than 3 months with no loss in activity. For peptide loading, HLA-Ig coated aAPC were washed twice with PBS and adjusted to 10⁷ beads/ml in 30 mg/ml of the peptide (final concentration). aAPC beads were stored in the peptide solution at 4°C.

*In vitro generation of dendritic cells.* Monocytes were isolated from PBMC by CD14⁺ magnetic separation (Miltenyi, Auburn, CA). The CD14⁺ cells were cultured in M' medium with 2% autologous serum, 100 ng/ml human GM-CSF, 50 ng/ml IL-4, and 5 ng/ml TGF-β1. After 5-7 days of culture, a maturation cocktail containing 10 ng/ml TNF-β and IL-1β, 1000 U/ml IL-6 (BD-Pharmingen, San Diego, CA) and 1 mg/ml PGE₂ (Sigma, St. Louis, MO) was added for 24 h. Cells displayed typical cell surface markers of DC (CD1a⁺, CD14⁻, CD86⁺). For peptide loading, DC were harvested and incubated with 30 mg/ml in M' medium at a density of 1-2 x 10⁶ cells/ml.

*In vitro CTL induction*. CD8⁺ T lymphocytes were enriched from PBMC by depletion of CD8⁻ cells using a CD8 isolation kit (Miltenyi, Auburn, CA). The resulting population, comprising more than 90% CD8⁺ T cells, was used as responder cells and stimulated with either peptide-pulsed DC or with peptide-pulsed aAPC. Ten thousand responder cells/well were cocultured with either 5 x 10³ DC/well or 10⁴ peptide-pulsed aAPC/well in a 96-well round-bottom plate in 200 µl M' medium/well supplemented with 5% autologous plasma and 3% TCGF. No additional allogeneic feeder cells were used either for induction or for expansion of CTL. TCGF was prepared as described in Oelke et al., Clin. Canc. Res. 6, 1997-2005, 2000. Medium and TCGF was replenished twice a week. On day 7 and weekly thereafter, T cells were harvested, counted, and replated at 10⁴ T cells/well together with either 5 x 10³ peptide-pulsed autologous DC/well or 10⁴ peptide-pulsed aAPC/well in complete medium supplemented with 3% TCGF.

*Dimer staining and intracellular cytokine staining (ICS) analysis.* Cells were stained with FITC-conjugated CD8 mAb and Mart-1- or CMV-pulsed A2-Ig and in a second step with anti-mouse Ig-PE to detect the Ig-A2 dimer, as described in Greten et al., Proc. Natl. Acad. Sci. USA 95, 7568-73, 1998. For the control staining, A2-Ig was either loaded with an irrelevant peptide or unloaded, as indicated. Similar background staining was observed using either unloaded A2-Ig (as in Figure 2) or A2-Ig loaded with an irrelevant A2-binding peptide (as in Figure 4). For analysis, we gated on CD8⁺ cells.

ICS was performed as described (BD-Pharmingen, San Diego, CA- ICS protocol) with the following modifications. One million effector cells were stimulated for 5 h at 37°C with 2 x 10⁵ peptide-pulsed T2 cells (30 µg/ml) or 10⁶ melanoma cells. When melanoma cells were used as target, 0.5 ng/ml phorbol 12-myristate 13-acetate (PMA) and 4 ng/ml ionomycin were added. Control experiments revealed that low doses of PMA and ionomycin did not induce cytokine production in the effector cells. Intracellular staining was performed with FITC-labeled IFN-g or IL-4 mAbs (BD, San Diego, CA).

*⁵¹Cr-release assay*. ⁵¹Cr-release assays were performed as described in Oelke *et al*., 2000. CTL activity was calculated as the percentage of specific ⁵¹Cr release using the following equation: % specific killing = (sample release - spontaneous release) ÷ (maximal release - spontaneous release) x 100%.

### EXAMPLE 2

### Induction and Expansion of Mart-1- and CMV-Specific CTL by aAPC

This example demonstrates the induction and expansion of antigen-specific CTL by two clinically relevant targets, CMV-peptide pp65 and Mart-1. These peptides have widely varying affinities for their cognate TCR. The CMV-peptide pp65 is known to be a high affinity peptide, whereas the modified Mart-1 peptide, derived from a melanocyte self antigen, is a low affinity peptide (Valmori et al., Int. Immunol. 11, 1971-80, 1999).

Current approaches use autologous peptide-pulsed DC to induce antigen-specific CTL from normal PBMC (Figure 1). These approaches often use DC- or CD40L-stimulated autologous B cells to induce antigen-specific CTL over 2-4 stimulation cycles (Figure 1, Step 2) until the antigen-specific CTL become a prominent part of the culture. We, therefore, compared aAPC induction to induction by DC. T cells were isolated, purified, and induced with either Mart-1-loaded aAPC or monocyte-derived autologous DC-pulsed with Mart-1 peptide. CD8⁺ T cells were stimulated once a week with either the DC or aAPC for a total of three rounds.

In a representative experiment the total number of T cells increased from 1 x 10⁶ to 20 x 10⁶ in the DC-induced cultures and from 1 x 10⁶ to 14 x 10⁶ in the aAPC induced cultures. Antigenic specificity of the culture was analyzed after 3 weeks by both A2-Ig dimer staining and ICS. In our hands, ICS staining can be up to twice as sensitive as dimer staining, due possibly to heterogeneity in the induced CTL population. ICS will detect a broader population of high and low affinity CTL than dimer staining. Cells were stained with FITC-conjugated CD8 mAb and Mart-1-pulsed A2-Ig as described. For ICS cells were incubated with peptide-pulsed T2 cells in regular medium without cytokines. After 1 h, Monensin (Golgi-stop) was added to the culture. After 6h the T cells were harvested and analyzed by ICS. The percent of peptide-specific CD8⁺ CTL is shown in the upper right corner.

After 3 rounds of stimulation with MART-1 peptide-loaded aAPC, 62.3% of all CD8 CTL were Mart-1-specific, as determined by ^{Mart-1} A2-Ig dimer staining (Figure 2A, left hand side) and 84.3% as determined by intracellular cytokine staining (ICS) (Figure 2A, right hand side). Differences seen between HLA-Ig dimer staining and ICS analysis of antigen-specific CTL probably relate to the diversity in the TCR repertoire used by the DC or aAPC induced CTL populations. Heterogeneity in peptide induced antigen-specific CTL populations has been previously reported. Valmori et al., J. Immunol. 168, 4231-40, 2002. The diversity in the repertoire may relate to higher and lower affinity CTL induced that are recognized by one but not the other assay.

In contrast to the results obtained with aAPCs, autologous DC induced only 29.7% MART-1-specific cells by dimer staining and 61.1% by ICS Mart-1-specific CTL (Figure 2B).

To explore the growth potential of aAPC-stimulated PBMC, T cells were stimulated with aAPC for 7 weeks. Starting from 1 x 10⁶ total CD8⁺ T cells that were less than 0.05% Mart-1-specific, cells expanded to approximately 10⁹ CTL that were greater than 85% Mart-1-specific (Figures 2C and 2D). This represents at least a 10⁶-fold expansion of antigen-specific T cells in under two months.

aAPC-mediated stimulation was as effective as, if not better than, stimulation by DC for both Mart-1 and CMV-induced CTL (Table 1).

**Table 1.**

| Donor | Stimulus | Cytokine assay (% positive) | | | | Dimer staining (% positive) | |
|---|---|---|---|---|---|---|---|
| | | only T cells | T2 | CMV | MART-1 | unloaded A2-Ig | MART-1 loaded A2-Ig |
| 1A1 | DC | nd | nd | nd | nd | 0.1 | 13.5 |
| 1A1 | aAPC | nd | nd | nd | nd | 0.7 | 33.4 |
| 1A4 | DC | 0.2 | 0.6 | 0.4 | 13.2 | 1.5 | 14.4 |
| 1A4 | aAPC | 0.3 | 3.2 | 2.6 | 32.6 | 2 | 54 |
| 5A | DC | 0.2 | 0.2 | 0.2 | 49.1 | 0.8 | 19.2 |
| 5A | aAPC | nd | nd | nd | nd | 0.1 | 4 |
| 6A | DC | 0 | 0.1 | 0.1 | 68.7 | 0.1 | 20.8 |
| 6A | aAPC | 0 | 0 | 0 | 84.6 | 0.3 | 65.9 |
| 7A | DC | nd | nd | nd | nd | 2.9 | 28.0 |
| 7A | aAPC | nd | nd | nd | nd | 0.2 | 79.5 |
| | | | | | | unloaded A2-Ig | CMV loaded A2-Ig |
| 2B1 | DC | nd | nd | nd | nd | 1.7 | 58 |
| 2B1 | aAPC | nd | nd | nd | nd | 2.6 | 69 |
| 8B | DC | nd | nd | nd | nd | 1.2 | 83.8 8 |
| 8B | aAPC | nd | nd | nd | nd | 4.7 | 88.1 |
| 9B | DC | 0.2 | nd | 93 | 0.2 | 2.3 | 98.5 |
| 9B | aAPC | 0.3 | 0.3 | 82 | 0.2 | 0.6 | 92.1 |

This was seen in four of five experiments using cells from three different healthy donors and a patient with metastatic melanoma (for Mart-1-loaded aAPC) and cells from three different donors (for CMV-loaded aAPC). For Mart-1 induction, aAPC induced about 2-3 fold more antigen-specific cells than with DC, as seen both by HLA-Ig dimer staining and ICS. This was also seen with a patient with metastatic melanoma. Induction of CMV-specific CTL was more robust than Mart-1-specific CTL; even after a single round of stimulation with aAPC up to 90% of the CTL population were CMV-specific. Slightly fewer CMV-specific CTL were obtained using DC. Thus, aAPC were generally as effective as, if not better than, DC at inducing antigen-specific CTL in two different CTL systems from multiple healthy donors, as well as a patient with melanoma.

aAPC also mediated significant expansion of CTL-specific for the A2-restricted subdominant melanoma epitope NY-ESO-1 (Jager et al., J. Exp. Med. 187, 265-70, 1998) and the subdominant EBV epitope derived from LMP-2 (Lee et al., J. Virol. 67, 7428-35, 1993) (see Table 2). Approximately 1.2% of all CD8⁺ cells were NY-ESO-1-specific after three rounds of aAPC stimulation. While this is clearly lower than seen in expansion of CTL that are specific for immunodominant epitopes, lower numbers of antigen specific CTL are expected when analyzing expansion of CTL specific for subdominant epitopes.

NY-ESO-1-specific CTL mediated lysis of cognate specific target cells but not irrelevant target cells (Table 2). In these experiments, CTL were cultured for 3-4 weeks before analysis. The frequency of antigen-specific CTL was analyzed by dimer staining for the LMP-2-specific CTL or by tetramer staining and ⁵¹Cr release assay for the NY-ESO-1-specific CTL. Table 2 shows the percent specific lysis observed at an E:T ratio of 25:1 and the percent of peptide-specific, CD8⁺ T cells as determined by flow cytometry using either A2-Ig dimer or tetramer. In contrast, DC-based stimulation resulted in a significantly lower frequency of NY-ESO-1-specific CTL without detectable cytotoxic activity in a standard ⁵¹Cr release assays.

**Table 2.**

| Donor | Stimulus | Cytotoxicity assay (% lysis) | | Staining (% positive) | |
|---|---|---|---|---|---|
| | | T2 + Mart-1 | T2 + NY-ESO | HIV tetramer | NY-ESO tetramer |
| 8C1 | DC | 17.9 | 20.2 | 0.3 | 0.6 |
| 8C1 | aAPC | 4.3 | 16.9 | 0.2 | 1.2 |
| | | | | unloaded A2-Ig | LMP-2 loaded A2-Ig |
| 4D1 | DC | nd | nd | 0.3 | 0.5 |
| 4D1 | aAPC | nd | nd | 5.0 | 24.7 |

### EXAMPLE 3

### Recognition of Endogenously Processed Antigen by aAPC-Induced PBMC

A useful criterion in evaluating CTL function is the recognition of targets expressing endogenous antigen-HLA complexes. Initial work using peptide-pulsed DC for expansion of melanoma-specific CTL resulted in low affinity CTL that mediated lysis of targets pulsed by the cognate antigen but often did not recognize melanoma targets that endogenously expressed antigen-HLA complexes. Yee et al., J. Immunol. 162, 2227-34, 1999. We therefore studied the ability of aAPC-induced CTL to recognize endogenous Mart-1 or pp65 CMV antigen (Figure 3).

For the ICS staining the cells were incubated with target cells in regular medium without cytokines. To increase the sensitivity of the ICS assay, a low dose of PMA and ionomycin was added to the medium. As described in Perez-Diez et al., Cancer Res. 58, 5305-09, 1998, this approach enabled us to detect more antigen specific T cells in the population. Differences in the results with or without this additional stimulation were dependent on the stimulus. The enhancement seen with low dose PMA and ionomycin was more prominent when allogenic tumor cells were used as stimulator cells (up to 3-4 fold) but was insignificant when peptide-pulsed T2 cells or A293 cells were used to stimulate the antigen-specific T cells. The addition of low dose of PMA and ionomycin did not change background activity, as can be seen in Figures 3A and 3C. Classic chromium release lysis assays were performed without addition of PMA and ionomycin (Figures 3B and 3D).

When Mart-1-specific aAPC-induced cells were stimulated with melanoma target cells, approximately 37% produced IFN-γ (Figure 3A). A comparable number produced IL-4 (Figure 5). A control Mart-1⁺/HLA-A2⁻ melanoma target did not stimulate significant effector cytokine production. Furthermore, aAPC-induced effector CTL populations mediated dose-dependent lysis of target Mart-1⁺/HLA-A2⁺ melanoma target cells but not control Mart-1⁺/HLA-A2⁻ targets (Figure 3B). aAPC-induced Mart-1-specific CTL derived from PBMC obtained from a patient with advanced melanoma were also able to mediate lysis of an HLA-A2⁺ Mart-1 expressing melanoma cells, with a 14.7% lysis seen at an E:T ratio of 25:1.

aAPC were also able to induce CMV-specific CTL that recognized endogenously processed and presented pp65 antigen (Figures 3C and 3D). When stimulated with A293-N pp65⁺ targets (A293 cells transfected with pp65), approximately 45% of the cells produced IFNγ. aAPC-induced effector CTL populations also mediated dose-dependent lysis of transfected target cells but not control targets (Figure 3D). Thus aAPC-induced CTL cultures from both normal healthy donors as well as from patients with melanoma recognized endogenously processed antigen-HLA complexes.

A portion of antigen-specific CTL produced either or both IFN-γ and IL4, whether induced by aAPC or DC (Figure 5). Human CD8⁺ cells producing both IFN-γ and IL4 have been reported in DC-based *ex vivo* expansion. Oelke *et al.,* 2000. Our results with aAPC confirm those interesting DC-based findings and show that aAPC-mediated stimulation results in phenotypically similar antigen-specific CTL.

### EXAMPLE 4

### Expansion of CMV-Specific CTL by aAPC

One limitation associated with use of DC is that expansion of CTL to clinically relevant numbers requires either leukapheresis to obtain enough DC or use of a non-specific expansion such as anti-CD3 beads (see Figure 1, Step 3). We therefore compared the "expansion" phase using aAPC or anti-CD3/anti-CD28-beads. During the expansion of CMV-specific CTL, there was a 7-fold increase in the total number of CTL using anti-CD3/anti-CD28 beads. However, the percentage of antigen-specific cells declined from 87.9% to 7.3% (compare Figures 4A and 4B). This problem has limited the usefulness of using anti-CD3 based expansion of diverse CTL populations. Maus et al., Nature Biotechnol. 20, 143-48, 2002. In contrast, when CMV-specific aAPC were used to expand antigen-specific CTL there was no concomitant loss of antigenic specificity. The percentage of CMV-specific CTL remained over 86% in those cultures, and there was still a 7-fold increase in the number of T cells (compare Figures 4A and 4C). Thus, HLA-Ig-based aAPC support continued expansion of CTL in an antigen-specific fashion and represent a significant advance over anti-CD3 based expansion.

## Claims

1. A rigid solid support, comprising:
(A) an antibody that specifically binds to CD28; and
(B) an MHC class I immunoglobulin complex comprising:
(1) two fusion proteins, wherein each fusion protein comprises an MHC class I α chain comprising a peptide binding groove, and an immunoglobulin heavy chain comprising a variable region, wherein the immunoglobulin heavy chain is C-terminal to the MHC chain, and wherein the immunoglobulin heavy chains of the two fusion proteins associate to form the MHC class I immunoglobulin complex, wherein the MHC class I immunoglobulin complex comprises a first MHC class I peptide binding cleft and a second MHC class I binding cleft;
(2) two MHC class I β₂ microglobulin polypeptides; and
(3) two immunoglobulin light chains.

2. The rigid solid support of claim 1, wherein the rigid solid support is a particle.

3. The rigid solid support of claim 2, wherein the particle is an artificial particle.

4. The rigid solid support of claim 3, wherein the artificial particle is a bead.

5. The rigid solid support of claim 1 wherein the MHC class I immunoglobulin complex comprises an antigenic peptide.

6. The rigid solid support of claim 5 wherein the antigenic peptide is selected from the group consisting of a peptide of a tumor-associated antigen, a peptide of an autoantigen, a peptide of an alloantigen, and a peptide of an infectious agent antigen.

7. The rigid solid support of claim 1 comprising at least two MHC class I immunoglobulin complexes.

8. The rigid solid support of claim 7 wherein an identical antigenic peptide is bound to each peptide binding groove of the at least two MHC class I immunoglobulin complexes.

9. The rigid solid support of claim 7 wherein different antigenic peptides are bound to each peptide binding groove of the at least two MHC class I immunoglobulin complexes.

10. A preparation comprising a plurality of the rigid solid supports according to any one of the preceding claims.

11. The preparation of claim 10 further comprising a pharmaceutically acceptable carrier.

12. A method of inducing the formation of antigen-specific T cells, comprising the step of:
contacting an isolated preparation comprising a plurality of precursor T cells with the rigid solid support of claim 1, wherein antigenic peptides are bound to the peptide binding grooves, thereby inducing members of the plurality of precursor T cells to form a first cell population comprising antigen-specific T cells that recognize the antigenic peptides, wherein the number or percentage of antigen-specific T cells in the first cell population is greater than the number or percentage of antigen-specific T cells that are formed if precursor T cells are incubated with a rigid solid support that comprises an antibody that specifically binds to CD3 but does not comprise the MHC class I immunoglobulin complex.

13. The method of claim 12 wherein the antigen-specific T cells are cytotoxic T cells.

14. The method of claim 12 wherein the antigen-specific T cells are helper T cells.

15. The method of claim 12 wherein the antigen-specific T cells are regulatory T cells.

16. The method of claim 12 further comprising the step of separating the antigen-specific T cells from the first cell population.

17. The method of claim 12 further comprising the step of incubating the first cell population with a second rigid solid support of claim 1, wherein antigenic peptides are bound to the peptide binding grooves and wherein the step of incubating is carried out for a period of time sufficient to form a second cell population comprising an increased number or percentage of antigen-specific T cells relative to the number or percentage of antigen-specific T cells in the first cell population.

18. The method of claim 12 wherein the antigenic peptides are identical.

19. The method of claim 12 wherein the antigenic peptides are different.

20. The method of claim 12 wherein the isolated preparation is contacted with two solid supports, wherein different antigenic peptides are bound to the peptide binding grooves of the MHC class I immunoglobulin complexes of each of the solid supports.

21. An *in vitro* method of increasing the number or percentage of antigen-specific T cells in a population of cells, comprising the step of:
incubating a first cell population comprising antigen-specific T cells with the rigid solid support of claim 1, wherein antigenic peptides are bound to the peptide binding grooves, wherein the step of incubating is carried out for a period of time sufficient to form a second cell population comprising an increased number or percentage of antigen-specific T cells relative to the number or percentage of antigen-specific T cells in the first cell population.

22. The method of claim 21 wherein the first cell population is a homogeneous cell population.

## Patentansprüche

1. Starrer fester Träger, umfassend:
(A) einen Antikörper, der spezifisch an CD28 bindet; und
(B) einen MHC-Klasse-I-Immunglobulinkomplex umfassend:
(1) zwei Fusionsproteine, wobei jedes Fusionsprotein eine eine peptidbindende Furche umfassende MHC-Klasse-I-α-Kette und eine eine variable Region umfassende schwere Immunglobulinkette umfasst, wobei sich die schwere Immunglobulinkette C-terminal zur MHC-Kette befindet, und wobei die schweren Immunglobulinketten der beiden Fusionsproteine assoziieren, um den MHC-Klasse-I-Immunglobulinkomplex zu bilden, wobei der MHC-Klasse-I-Immunglobulinkomplex eine erste MHC-Klasse-I-Peptidbindungsspalte und eine zweite MHC-Klasse-I-Bindungsspalte umfasst;
(2) zwei MHC-Klasse-I-β₂-Mikroglobulinpolypeptide; und
(3) zwei leichte Immunglobulinketten.

2. Starrer fester Träger nach Anspruch 1, wobei der starre feste Träger ein Partikel ist.

3. Starrer fester Träger nach Anspruch 2, wobei das Partikel ein künstliches Partikel ist.

4. Starrer fester Träger nach Anspruch 3, wobei das künstliche Partikel eine Perle ist.

5. Starrer fester Träger nach Anspruch 1, wobei der MHC-Klasse-I-Immunglobulinkomplex ein antigenes Peptid umfasst.

6. Starrer fester Träger nach Anspruch 5, wobei das antigene Peptid ausgewählt ist aus der Gruppe bestehend aus einem Peptid eines tumorassoziierten Antigens, einem Peptid eines Autoantigens, einem Peptid eines Alloantigens und einem Peptid eines Infektionserregerantigens.

7. Starrer fester Träger nach Anspruch 1, umfassend wenigstens zwei MHC-Klasse-I-Immunglobulinkomplexe.

8. Starrer fester Träger nach Anspruch 7, wobei an jede peptidbindende Furche der wenigstens zwei MHC-Klasse-I-Immunglobulinkomplexe ein identisches antigenes Peptid gebunden ist.

9. Starrer fester Träger nach Anspruch 7, wobei an jede peptidbindende Furche der wenigstens zwei MHC-Klasse-I-Immunglobulinkomplexe unterschiedliche antigene Peptide gebunden sind.

10. Präparat, umfassend eine Mehrzahl der starren festen Träger nach einem der vorhergehenden Ansprüche.

11. Präparat nach Anspruch 10, ferner umfassend einen pharmazeutisch verträglichen Trägerstoff.

12. Verfahren zum Induzieren der Bildung antigenspezifischer T-Zellen, umfassend den Schritt:
In-Kontakt-bringen eines isolierten Präparats, das eine Mehrzahl an Vorläufer-T-Zellen umfasst, mit dem starren festen Träger nach Anspruch 1, wobei antigene Peptide an die peptidbindenden Furchen gebunden werden, wodurch Mitglieder der Mehrzahl an Vorläufer-T-Zellen zur Bildung einer ersten Zellpopulation induziert werden, die antigenspezifische T-Zellen umfasst, die die antigenen Peptide erkennen, wobei die Anzahl oder der Prozentsatz antigenspezifischer T-Zellen in der ersten Zellpopulation größer ist als die Anzahl oder der Prozentsatz antigenspezifischer T-Zellen, die gebildet werden, wenn Vorläufer-T-Zellen mit einem starren festen Träger inkubiert werden, der einen Antikörper umfasst, der spezifisch an CD3 bindet, aber keinen MHC-Klasse-I-Immunglobulinkomplex umfasst.

13. Verfahren nach Anspruch 12, wobei die antigenspezifischen T-Zellen zytotoxische T-Zellen sind.

14. Verfahren nach Anspruch 12, wobei die antigenspezifischen T-Zellen T-Helferzellen sind.

15. Verfahren nach Anspruch 12, wobei die antigenspezifischen T-Zellen Regulator-T-Zellen sind.

16. Verfahren nach Anspruch 12, ferner umfassend den Schritt des Abtrennens der antigenspezifischen T-Zellen von der ersten Zellpopulation.

17. Verfahren nach Anspruch 12, ferner umfassend den Schritt des Inkubierens der ersten Zellpopulation mit einem zweiten starren festen Träger nach Anspruch 1, wobei antigene Peptide an die peptidbindenden Furchen gebunden werden und wobei der Inkubationsschritt über einen Zeitraum durchgeführt wird, der zur Bildung einer zweiten Zellpopulation ausreicht, die im Vergleich mit der Anzahl oder dem Prozentsatz antigenspezifischer T-Zellen in der ersten Zellpopulation eine erhöhte Anzahl oder einen erhöhten Prozentsatz antigenspezifischer T-Zellen umfasst.

18. Verfahren nach Anspruch 12, wobei die antigenen Peptide identisch sind.

19. Verfahren nach Anspruch 12, wobei die antigenen Peptide unterschiedlich sind.

20. Verfahren nach Anspruch 12, wobei das isolierte Präparat mit zwei festen Trägern in Kontakt gebracht wird, wobei unterschiedliche antigene Peptide an die peptidbindenden Furchen der MHC-Klasse-I-Immunglobulinkomplexe von jedem der festen Träger gebunden werden.

21. *In vitro*-Verfahren zum Erhöhen der Anzahl oder des Prozentsatzes antigenspezifischer T-Zellen in einer Population von Zellen, umfassend den Schritt:
Inkubieren einer ersten Zellpopulation, die antigenspezifische T-Zellen umfasst, mit dem starren festen Träger nach Anspruch 1, wobei antigene Peptide an die peptidbindenden Furchen gebunden werden, wobei der Inkubationsschritt über einen Zeitraum durchgeführt wird, der zur Bildung einer zweiten Zellpopulation ausreicht, die im Vergleich mit der Anzahl oder dem Prozentsatz antigenspezifischer T-Zellen in der ersten Zellpopulation eine erhöhte Anzahl oder einen erhöhten Prozentsatz antigenspezifischer T-Zellen umfasst.

22. Verfahren nach Anspruch 21, wobei die erste Zellpopulation eine homogene Zellpopulation ist.

## Revendications

1. Support solide rigide, comprenant :
(A) un anticorps qui se lie spécifiquement au CD28 ; et
(B) un complexe immunoglobuline CMH de classe I comprenant :
(1) deux protéines de fusion, où chaque protéine de fusion comprend une chaine α de CMH de classe I comprenant un sillon de liaison des peptides et une chaîne lourde d'immunoglobuline comprenant une région variable, où la chaîne lourde d'immunoglobuline est C-terminale par rapport à la chaîne du CMH, et où les chaînes lourdes d'immunoglobuline des deux protéines de fusion s'associent pour former le complexe CMH de classe I immunoglobuline, où le complexe immunoglobuline CMH de classe I comprend une première fente de liaison de peptide du CMH de classe I et une seconde fente de liaison de CMH de classe I ;
(2) deux polypeptides de β₂-microglobuline du CMH de classe I ; et
(3) deux chaînes légères d'immunoglobuline.

2. Support solide rigide selon la revendication 1, où le support solide rigide est une particule.

3. Support solide rigide selon la revendication 2, où la particule est une particule artificielle.

4. Support solide rigide selon la revendication 3, où la particule artificielle est une bille.

5. Support solide rigide selon la revendication 1, où le complexe immunoglobuline CMH de classe I comprend un peptide antigénique.

6. Support solide rigide selon la revendication 5, où le peptide antigénique est choisi dans le groupe constitué d'un peptide d'un antigène associé à une tumeur, d'un peptide d'un auto-antigène, d'un peptide d'un allo-antigène et d'un peptide d'un antigène d'un agent infectieux.

7. Support solide rigide selon la revendication 1, comprenant au moins deux complexes immunoglobuline CMH de classe 1.

8. Support solide rigide selon la revendication 7, où un peptide antigénique identique est lié à chaque sillon de liaison des peptides des au moins deux complexes immunoglobulines CMH de classe I.

9. Support solide rigide selon la revendication 7, où des peptides antigéniques différents sont liés à chaque sillon de liaison des peptides des au moins deux complexes immunoglobulines CMH de classe I.

10. Préparation comprenant une pluralité de supports solides rigides selon l'une quelconque des revendications précédentes.

11. Préparation selon la revendication 10, comprenant en outre un vecteur pharmaceutiquement acceptable.

12. Procédé d'induction de la formation de cellules T spécifiques de l'antigène, comprenant l'étape consistant à :
mettre en contact une préparation isolée comprenant une pluralité de cellules T précurseurs avec le support solide rigide selon la revendication 1, où les peptides antigéniques sont liés aux sillons de liaison des peptides, induisant de cette manière des membres de la pluralité des cellules T précurseurs pour former une première population cellulaire comprenant des cellules T spécifiques de l'antigène qui reconnaissent les peptides antigéniques, où le nombre ou le pourcentage des cellules T spécifiques de l'antigène dans la première population cellulaire est supérieur au nombre ou au pourcentage des cellules T spécifiques de l'antigène qui sont formées si les cellules T précurseurs sont incubées avec un support solide rigide qui comprend un anticorps qui se lie spécifiquement au CD3 mais qui ne comprend pas le complexe immunoglobuline CMH de classe I.

13. Procédé selon la revendication 12, dans lequel les cellules T spécifiques de l'antigène sont des cellules T cytotoxiques.

14. Procédé selon la revendication 12, dans lequel les cellules T spécifiques de l'antigène sont des cellules T auxiliaires.

15. Procédé selon la revendication 12, dans lequel les cellules T spécifiques de l'antigène sont des cellules T régulatrices.

16. Procédé selon la revendication 12, comprenant en outre l'étape consistant à séparer les cellules T spécifiques de l'antigène issues de la première population cellulaire.

17. Procédé selon la revendication 12, comprenant en outre l'étape consistant à incuber la première population cellulaire avec un second support solide rigide selon la revendication 1, où les peptides antigéniques sont liés aux sillons de liaison des peptides et où l'étape d'incubation est réalisée pendant une période de temps suffisante pour former une seconde population cellulaire comprenant un nombre ou un pourcentage accru de cellules T spécifiques de l'antigène par rapport au nombre ou au pourcentage de cellules T spécifiques de l'antigène dans la première population cellulaire.

18. Procédé selon la revendication 12, dans lequel les peptides antigéniques sont identiques.

19. Procédé selon la revendication 12, dans lequel les peptides antigéniques sont différents.

20. Procédé selon la revendication 12, dans lequel la préparation isolée est mise en contact avec deux supports solides, où des peptides antigéniques différents sont liés aux sillons de liaison des peptides des complexes immunoglobulines CMH de classe I de chacun des supports solides.

21. Procédé *in vitro* permettant d'augmenter le nombre ou le pourcentage des cellules T spécifiques de l'antigène dans une population de cellules, comprenant l'étape consistant à :
incuber une première population cellulaire comprenant des cellules T spécifiques de l'antigène avec le support solide rigide selon la revendication 1, où les peptides antigéniques sont liés aux sillons de liaison des peptides, où l'étape d'incubation est réalisée pendant une période de temps suffisante pour former une seconde population cellulaire comprenant un nombre ou un pourcentage accru de cellules T spécifiques de l'antigène par rapport au nombre ou au pourcentage de cellules T spécifiques de l'antigène dans la première population cellulaire.

22. Procédé selon la revendication 21, dans lequel la première population cellulaire est une population de cellules homogènes.
